Europäisches Patentamt

European Patent Office

Office européen des brevets

(19)

(11) Publication number: **0 125 468 B1**

## EUROPEAN PATENT SPECIFICATION

(12)

(45) Date of publication of patent specification: **28.10.92**

(51) Int. Cl.⁵: **C12N 15/03**, C05F 11/08, A01N 63/00, A01C 1/06, A01H 17/00

(21) Application number: **84103792.2**

(22) Date of filing: **05.04.84**

(54) **Agricultural-chemical-producing endosymbiotic bacteria and method of preparing and using same.**

(30) Priority: **13.04.83 US 484560**
**20.09.83 US 534071**

(43) Date of publication of application:
**21.11.84 Bulletin 84/47**

(45) Publication of the grant of the patent:
**28.10.92 Bulletin 92/44**

(84) Designated Contracting States:
**AT BE CH DE FR GB IT LI LU NL SE**

(56) References cited:
**EP-A- 0 064 720**
**FR-A- 2 355 453**
**FR-A- 2 386 504**
**FR-A- 2 396 082**

**BIOLOGICAL ABSTRACTS, vol. 75, 1983, Biosciences Information Service Philadelphia, page 588, ref. 5737; O.-Y. DU et al.: "A preliminary report on the establishment of nitrogen fixation system in the crown gall of tomato(lycopersicon esculentum)plant", & ACTA BOT SIN 23(6), 453-458**

(73) Proprietor: **CROP GENETICS INTERNATIONAL CORPORATION**
**7170 Standard Drive**
**Hanover, MD 21076(US)**

(72) Inventor: **Carlson, Peter Spikins**
**4418 Garrison Street, N.W.**
**Washington, D.C. 20016(US)**

(74) Representative: **Patentanwälte Grünecker, Kinkeldey, Stockmair & Partner**
**Maximilianstrasse 58**
**W-8000 München 22(DE)**

NATURE, vol. 252, 29th November 1974, pages 393-395; P.S. CARLSON et al.: "Forced association between higher plant and bacterial cells in vitro"

CHEMICAL ABSTRACTS, vol. 89, no. 3, 17th July 1978, page 187, no. 18437v, Columbus, Ohio, US; P.S. CARLSON et al.: "Novel cellular associations formed in vitro" & MOL. GENET. MODIF. EUCARYOTES, (PROC. ANNU. WORKSHOPS) 1974-1975 (PUB. 1977), 43-56

SCIENCE, vol. 219, 11th February 1983, pages 671-676; K.A. BARTON et al.: "Prospects in plant genetic engineering"

NATURE, vol. 276, 30th November 1976, pages 498-500, Macmillan Journals Ltd.; V.A. SMITH et al.: "Effect of agrocin 84 on attachment of agrobacterium tumefaciens to cultured tobacco cells"

BIOLOGICAL ABSTRACTS, vol. 62, 1976, Biosciences Information Service Philadelphia, ref. 2060; M. ONDREJ et al.: "Experimental proposals for the transfer of genes regulating atmospheric nitrogen fixation to cereals" & BIOL LISTY 40(3): 214-216. 1975

## Description

The present invention relates to agricultural-chemical-producing bacteria, particularly agricultural-chemical-producing bacteria capable of entering into non-pathogenic endosymbiotic relationships with host plants whereby the bacteria provide some or all of the plant's agricultural-chemical requirements.

Modern commerical agriculture is heavily dependent on the use of agricultural chemicals to enhance the performance of plants. Most notably, chemical fertilizers, particularly fixed-nitrogen fertilizers, are the most common limiting nutrient in crop productivity and often are the most expensive single input for the farmer. Other agricultural chemicals are widely used at great expense to kill plant pests, to prevent disease, or to improve the growing environment of the plant. Still other agricultural chemicals may be added to growing plants or harvested crops to diminish or enhance natural properties or to alter or improve the appearance or sensory appeal of the plant or crop.

Agricultural chemicals include, as well, natural or synthetic plant growth regulators, including e.g. hormones. Such chemicals are well known to those having ordinary skill in the agricultural art and are hereinafter generically referred to as "agricultural chemicals".

Microorganisms, such as bacteria, are a natural source of agricultural chemicals. For example, leguminous plants, such as soybeans, alfalfa and clover, derive some of their fixed nitrogen requirements through symbiotic relationships with bacteria of the genus Rhizobium. Particular species of Rhizobium infect the roots of leguminous plants forming nodules in which the bacteria are shielded from oxygen and provided with carbohydrate nutrients. In this anaerobic process, the rhizobia fix atmospheric nitrogen, which is then available for use by the plant.

Bacteria have also become sources of agricultural chemicals synthesised or prepared by man and applied by e.g. spraying to fields, growing plants, or harvested crops. Antifungal antibiotics, antibacterial antibiotics and insecticidal antibiotics, for example, have been produced by various bacteria. Antifungal antibiotics include blasticidin S which is produced by Streptomyces griseochromogenus, kasugamycin which is produced by Streptomyces kasugaensis, and polyoxins which are produced by Streptomyces cacaoi var. asoensis. Antibacterial antibiotics include streptomycin produced by Streptomyces griseus, and tetracylin produced by Streptomyces viridifaciens. Insecticidal antibiotics include tetranactin, produced by Streptomyces aureus strain S-3466, and the $\beta$- and $\delta$-endotoxins, produced by Bacillus thuringiensis. See K. Aizawa, "Microbial Control of Insect Pests", and T. Misato and K. Yoneyama, "Agricultural Antibiotics" in Advances in Agricultural Microbiology, N.S.S. Rao, Editor (1982).

Similarly, microorganisms have become sources of antibiotics for herbicide use. For example, cycloheximide, produced by Streptomyces griseus, and herbicidin A and B, produced by Streptomyces saganoensis, exhibit herbicidal activity. See Y. Sekizawa and T. Takematsu, "How to Discover New Antibiotics for Herbicidal Use" in Pesticide Chemistry, J. Miyamoto and P.C. Kearney, Editors (1983).

Moreover, microorganisms are known to produce a plant growth regulating substances such as various vitamins, auxins, cytokinins, gibberellin-like substances and other stimulating or inhibiting substances. Brown, in her work on "Seed and Root Bacterization" cited below, attributes the production of such substances to species of Azotobacter, Pseudomonas and Bacillus, including B. megaterium and B. subtilis.

Microorganisms have also been found to produce antiviral antibiotics, including laurusin, which has been isolated from Streptomyces lavendulae, and miharamycin, which has been isolated from Streptomyces miharuensis. See T. Misato, K. Ko, and I. Yamaguchi, "Use of Antibiotics and in Agriculture" in Advances in Applied Microbiology, Vol. 21, (1977).

Other types of bacteria are known to have the ability to solubilize phosphenate that is otherwise unavailable to plants. These include those bacteria belonging to the genera Bacillus and Pseudomonas. Attempts to utilize this ability of these bacteria by inoculating seeds or plants with the bacteria have produced inconsistent results. See, N.S.S. Rao, "Phosphate Solubilization by Soil Microorganisms" in Advance in Agricultural Microbiology, N.S.S. Rao, Editor (1982).

Few symbiotic associations between agricultural-chemical-producing bacteria and major crop species, such as wheat and corn, have been described in the scientific literature. The development of symbiotic relationships between such bacterial species and various crops would have many advantages over man-made synthesis and application of the chemical. The advantages of such associations are particularly apparent in the area of fixed-nitrogen fertilizers, which are hereinafter discussed in detail as exemplary of the advantages attainable through the practice of the present invention. It should be understood, however, that the teachings contained herein regarding bacterial production of fixed-nitrogen fertilizers may be extrapolated by those having ordinary skill in the art to other bacterially produced agricultural chemicals, such as those discussed above.

It is estimated that development of a symbiotic relationship between nitrogen-fixing bacteria and major crop species capable of providing even a minor fraction, e.g., 10-20%, of the fixed nitrogen requirements of non-leguminous crop plants, would produce an economically important substitution of photosynthetically-derived energy for fossil fuel energy now expended for production of nitrogen fertilizer. Currently, the major sources of fixed nitrogen for non-leguminous economic crop plants are man-made products such as anhydrous ammonia, inorganic nitrogen salts and synthetic organic nitrogen compounds. Present annual consumption of nitrogen fertilizer in the United States is estimated to be 12 million tons. Such nitrogen fertilizers are largely prepared by energy intensive processes for the conversion of atmospheric nitrogen into ammonia.

A major thrust of nitrogen-fixation research in recent years has been directed towards the isolation, characterisation, transformation and expression in plants of the nitrogen-fixing genes from rhizobia and other nitrogen-fixing bacteria by genetic modification of the plants themselves. This technique requires a complete understanding of the genetics and biochemistry of nitrogen fixation which, it is estimated, is 10 to 15 years or more in the future. See generally, J.E. Beringer, "Microbial Genetics and Biological Nitrogen Fixation", in Advances In Agricultural Microbiology, N.S.S. Rao, Editor (1982), and G.P. Roberts et al., "Genetics and Regulation of Nitrogen Fixation", Ann. Rev. Microbiol., 35:207-35 (1981).

Other types of bacteria are known to have the capability of fixing atmospheric nitrogen. These include those belonging to the genera Azotobacter, Azomonas, Derxia and Beijerinckia, which fix nitrogen aerobically, and those belonging to the genus Klebsiella which, like Rhizobium, fix nitrogen anaerobically. The literature is filled with reports of unsuccessful attempts to develop symbiotic relationships, similar to Rhizobium-legume relationships between these bacteria and non-leguminous plants.

In the early 1940's, for example, it was proposed in the Soviet Union and elsewhere that nitrogen-fixing bacteria could be used as soil inoculants to provide some or all of the fixed nitrogen requirements of non-luguminous crop plants. Indeed, claims of increased crop yield associated with such inoculations were widespread. Critical analysis of the early yield results, however, revealed that positive significant effects occured in only about one-third of the trials. It was later established that yield increases associated with bacterial inoculation, including inoculation with species of Azotobacter, were primarily due to changes in the rhizosphere microbial population, disease suppression by the inoculants, production of plant growth-promoting substances and mineralization of soil phosphates. No ability to establish an associative symbiotic relationship with nitrogen-fixing bacteria in non-leguminous plants had been demonstrated. See M.E. Brown, "Seed and Root Bacterization", Annual Review of Phytopathology, 12:181-197 (1974).

It has been demonstrated that an artificial symbiosis can be created between non-leguminous plant cells in tissue culture and aerobic nitrogen-fixing bacteria such as Azotobacter vinelandii. The demonstration involved forced association between higher plant and bacterial cells and did not provide a technique whereby a symbiotic association between such nitrogen-fixing bacteria and growing plants could be created. See P.S. Carlson, et al., "Forced Association Between Higher Plant and Bacterial Cells In Vitro", Nature, Volume 252, No. 5482, pp. 393-395 (1974).

Another effort to create higher organisms capable of fixing their own nitrogen, for example, involved attempts to create new organelles in higher organisms by similar forced association of lower forms of organisms having nitrogen-fixing abilities. Such efforts to parallel the theoretical biological development of known organelles have included, for example, the efforts of Burgoon and Bottino to induce the uptake of nitrogen-fixing Gloecapsa sp. blue-green algae by plant cells, the efforts of Davey and Cocking to induce the uptake of nitrogen-fixing Rhizobium sp. bacteria into plant cells, and the efforts of Giles to induce uptake of nitrogen-fixing Azotobacter vinelandii bacteria by ectomycorrhizal fungi. See A.C. Burgoon and P.J. Bottino, Journal of Heredity, Volume 67, pp. 223-226 (1972); M.R. Davey and E.C. Cocking, Nature, Volume 239, pp. 455-456 (1972); and K.L. Giles, "The Transfer of Nitrogen-Fixing Ability to Non-leguminous Plants", Plant Cell and Tissue Culture Principles and Applications, W.R. Sharp et al., Editors (1979). Neither the work of Burgoon and Bottino nor the work of Davey and Cocking was successful in generating a nitrogen-fixing higher organism. If the effects of Giles were successful, which has not been conclusively established, they would at best result in fungi with organelle-like structures capable of fixing nitrogen which are capable only of living on the outsides of roots of forest species rather than living endosymbiotically with major crop plants.

Another attempt to create a symbiotic relationship between nitrogen-fixing bacteria and non-leguminous plants involved the screening and selection of plant hybrids in an effort to identify some plant varieties which were capable of associating with aerobic nitrogen-fixing bacteria, such as Azotobacter vinelandii, in the soil. However, those efforts resulted in the identification of plant species which were only about .5% as active as soybean plants inoculated with rhizobia which were not deemed to be commercially useful. See S.W. Ela, et al., "Screening and Selection of Maize to Enhance Associative Bacterial Nitrogen Fixation", Plant Physiol., 70:1564-1567 (1982).

The numerous unsuccessful attempts to create associative symbiotic relationships between nitrogen-fixing bacteria and nonleguminous plants have resulted in the development of numerous techniques for genetic manipulation and analysis. These attemps have also resulted in rather extensive characterisation of the genetic makeup of nitrogen-fixing bacteria such as Azotobacter. For example, it has been discovered that mutant strains of Azotobacter vinelandii lacking the nitrogen-fixation gene could be transformed into bacteria having the capability to fix nitrogen by introduction of genetic material from Rhizobium species. See G.P. Roberts, "Genetics and Regulation of Nitrogen Fixation", Ann. Rev. Microbiol., 35:207-35 (1981). Moreover, new techniques for protoplast fusion, which involves promotion of interspecific genetic recombination by fusing two different cells which have had their cell walls removed, were recognised as providing a potentially attractive technique for the production of new strains for commerical purposes without requiring an understanding of the underlying genetics. It was acknoweledged in the scientific literature, however, that it has yet to be determined whether or not protoplast fusion can be used for genetic studies of gramnegative nitrogen-fixing bacteria. See. J.E. Beringer, "Microbial Genetics and Biological Nitrogen Fixation" in Advances in Agricultural Microbiology, N.S.S. Rao, Editor (1982) at 13.

Indeed, experimentation related to protoplast fusion of nitrogen-fixing bacteria has, prior to the present invention, failed to produce evidence that non-pathogenic endosymbiotic relationships between higher plants and fusion products of nitrogen fixing bacteria can be produced. For example, Du Qian-you and Fan Cheng-ying, "A Preliminary Report On the Establishment of Nitrogen Fixation System in the Crown Gall of Tomato Plant", Acta Botanica Sinica, Vol. 23, No. 6 (1981), described an attempt to effect protoplast fusion between parent strains of Agrobacterium tumefaciens (Pen$^r$, Ti) and Azotobacter chroococcum (Pen$^s$, Nif$^+$). The hybrids were selected for nitrogen fixation and their resistance to penecillin and were thereafter inoculated on the stem of a tomato plant. The inoculation produced crown galls or tumours found to possess nitrogenase activity under aerobic conditions according to the acetylene reduction test. The fusion products of the experiment produced a pathogenic response in the host plant and were not shown to be capable of entering into an endosymbiotic relationship with the plant. Specifically, it could not be ascertained whether the fixed nitrogen being produced in the vicinity of the tumours was being used by the plant as a source of nitrogen. Finally, it could not be determined whether the nitrogenase activity observed was the result of a pathogenic hybrid or of a penecillin resistant mutant of Azotobacter remaining in a mixed inoculum. Moreover, it has now been discovered that the attempts of Du Qian-you et al. to mimic the nodule formation mechanism of the Rhizobium-legume interaction by intentional inclusion of the tumour-forming Ti plasmid in their hybrids were headed in the opposite direction from that required to produce hybrid bacteria capable of entering into successful endosymbiotic relationships with plant hosts.

Other unsuccessful attempts to extend the range of nitrogen-fixing Rhizobium species include the insertion of the Ti plasmid from Agrobacterium tumefaciens into selected Rhizobium strains. See P.J.J. Hooykass, et al., J. Gen. Microbiology, 98:477-484 (1977). These studies resulted in Rhizobium strains able to produce galls on non-leguminous dicotyledenous plants which will fix nitrogen but only under anaerobic conditions not naturally occurring in such plants. The criticism outlined in the previous paragraph also applies to this work.

Despite substantial economic incentive to do so, the prior art has been unable to prepare hybrid nitrogen-fixing bacteria capable of entering into endosymbiotic relationships with non-leguminous plant hosts or any other hybrid agricultural-chemical-producing bacteria capable of entering into endosymbiotic relationships with plant hosts.

A method of producing hybrid agricultural-chemical-producing bacteria capable of entering into non-pathogenic, endosymbiotic relationships with a plant host has been discovered which comprises :

(A) in any convenient order:

(1) identifying said plant host;

(2) identifying an infecting bacterium which infects said plant host;

(3) selecting mutants of said infecting bacterium with one or more selectable traits in addition to ability to infect said host; and

(4) selecting an agricultural-chemical-producing bacterium having one or more selectable traits;

(B) forming fusion hybrids of said infecting bacterium and said agricultural-chemical-producing bacterium;

(C) selecting serially from the products of the previously performed step or substep, and in any convenient order:

(1) a bacterial subgroup comprising those hybrids having both at least one of said selectable traits of the agricultural-chemical-producing bacterium and at least one of said selectable traits of the infecting bacterium;

(2) a bacterial subgroup comprising those hybrids rich manifest the ability to interact with plant tissue

EP 0 125 468 B1

in the manner in which said infecting bacterium interacts with plant tissue during the initial phase of infection in said plant host;

(3) a bacterial subgroup comprising those hybrids which do not upon application to said host create manifestations of disease; and

(4) a bacterial subgroup comprising those hybrids having the ability to produce said agricultural chemical if not previously selected for; and

(D) selecting from the products of the last performed step of steps (C)(1) to (C)(4) those hybrids capable of improving the performance of said plant host when compared either by direct application of said agricultural chemical or by application of said agricultural-chemical-producing bacterium to said plant.

"Infecting bacteria" as used throughout this specification is intended to connote not only organisms which enter and live within the plant and normally produce symptoms of disease but also organisms which enter and live within the plant symbiotically or commensally. Indeed many of the plant infecting bacteria used in accordance with the present invention, while normally creating pathogenic responses in some plant hosts, will not normally produce such responses in all plant hosts. The term "bacteria" as used herein is intended to encompass bacteria, bacteria-like organisms and their equivalents, including gram positive bacteria, gram negative bacteria, actinomycetes and the like. The only limitations on the taxonomic classification of the organisms used in accordance with the present invention is that they be capable of protoplast fusion to produce viable hybrid organisms expressing phenotypical properties of both parents as more fully described herein.

The bacteria resulting from the process of the present invention may be introduced into crop plants by a variety of means, including injection, and may be employed to coat agricultural seed, to infect agricultural seed, in the preparation of a soil drench, and the like. The hybrid bacteria of the present invention, upon association with crop plants, are capable of supplying some or all of the plants' agricultural chemical needs. Agricultural chemicals which can be produced by hybrid bacteria in accordance with the present invention include fertilizers, particularly fixed-nitrogen fertilizers; antibiotics, including antibacterial, antiviral, antifungal, insecticidal, nematocidal, miticidal, and herbicidal agents; plant growth regulators.

The process is capable of producing agricultural-chemical-producing bacteria capable of entering into endosymbiotic relationships with both monocotyledonous and dicotyledonous plants. For diocotyledonous plants, infecting bacteria of the genus Agrobacterium are preferred, with strains of Agrobacterium tumefaciens being particularly preferred. Species of the genus Erwinia, such as Erwinia carotovora may also be used, as may species of the genus Pseudomonas, such as Pseudomonas solanacearum and Pseudomonas syringae, and of the genus Xanthomonas, such as Xanthomonas campestris. For monocotyledonous plants, species of the genus Erwinia, such as Erwinia stewartii, are preferred infecting bacteria. Species of the genus Xanthomonas, such as Xanthomonas campestris; species of the genus Azospirillum, such as Azospirillum lipoferum and Azospirillum brasilense; and species of the genus Pseudomonas, such as Pseudomonas syringae, are also contemplated as being useful. Pseudomonas syringae is contemplated as being particularly useful as an infecting bacterium for the formation of fusion products applicable to cereals, including temperate cereals and rice.

Preferred agricultural-chemical-producing bacteria and the chemicals and/or applications for which their metabolic products are useful are identified in Table I below and include organisms having the ability to produce fertilizers, including fixed nitrogen and chemicals capable of solubilising phosphates, antibiotics, including antibacterial compounds, antifungal compounds, antiviral compounds, insecticides, nematocides, miticides and herbicides, and plant growth regulators. Additionally, useful organisms may be selected or modified to produce other agricultural chemicals, as above defined, including fragrances and anti-feeding agents.

The processes of the present invention result in novel, stable fusion hybrid bacteria having the ability to produce one or more agricultural chemicals and enter into endosymbiotic relationships with a plant host. The nitrogen-fixing products of the present invention, for example, have been shown to improve the yield of non-leguminous crop plants growing under low fixed nitrogen conditions by amounts of from 10 to 180% due primarily, it is believed, to fixed nitrogen produced by the hybrids of nitrogen-fixing bacteria and infecting bacteria in the course of their endosymbiotic relationship with the plant host.

Additional objects and advantages of the invention will be set forth in part in the description which follows, and in part will be obvious from the description, or may be learned from the practice of the invention. The objects and advantages may be realised and attained by means of the instrumentalities and combinations particularly pointed out in the appended claims.

To achieve the objects and in accordance with the purpose of the invention, as embodied and broadly described herein, the hybrid fusion products of the above-described method are preferably the fusion products of bacteria having the same response to gram stain, preferably gram-negative bacteria. However,

6

certain gram-positive bacteria, notably members of the genus Bacillus, are also particularly useful for the formation of hybrid fusion products capable of producing agricultural chemicals. Moreover, the hybrid fusion products of the above-described method may be further modified by natural or artificial genetic techniques to improve their performance as sources of agricultural-chemical for the plant host. Such modification could result, for example, in the ability to excrete the agricultural chemical, such as fixed nitrogen, including the ability to excrete the agricultural chemical in a particular form, such as fixed nitrogen in the form of amino acids; the ability to continue production of the agricultural chemical even in the presence of adequate amounts of that chemical from other sources; in a reduction of the hybrid's resistance to cold temperatures (i.e., to prevent unintended proliferation of the hybrids from year to year); in enhancement of the hybrid's ability to withstand drought, disease or other physiological stress; in the introduction of additional agricultural-chemical-producing functions; or in modification of the hybrid so that it cannot grow outside the plant host.

While the steps of the above-described method may be carried out in any convenient order, it is desirable that the process of selection for agricultural-chemical-producing ability and for ability to interact with plant tissue in the manner in which the infecting bacterium interacts with plant tissue during the initial phases of infection in the host plant be carried out two or more times before the step relating to selection of those hybrids which do not manifest symptoms of disease in the plant host.

The selectable traits associated with the infecting bacterium may be e.g. antibiotic resistance, or heed for specific nutritional supplementation (auxotrophism). The selectable traits associated with the agricultural-chemical-producing bacterium may be the ability to produce an agricultural chemical alone or in combination with one or more of those traits previously mentioned with respect to the infecting bacterium. The interaction with plant tissue screened for in step C(2) of the above-described method may be, for example, the ability to bind to plant tissue or the ability to spread throughout the vascular system of the plant. Where the interaction associated with the initial phase of infection is binding to plant cells, it is desirable to limit further the bacterial subgroup resulting from step C prior to step D by selection of a bacterial subgroup for further screening in accordance with the above-described process comprising those hybrids which spread most readily throughout the plant host.

The fusion products are formed by protoplast or spheroplast fusion. By appropriate selection of the infecting bacterial component of the hybrid, fusion hybrids may be obtained which are capable of entering into endosymbiotic relationships with cereals, such as wheat, triticale, barley, rye, rice and oats; grasses such as brome grass, blue grass, tall fescue grass and bermuda grass; tropical grasses, such as sugar cane, corn, millet and sorghum; solanaceous plants, such as potatoes, tomatoes, tobacco, eggplant and pepper; brassicaceous plants such as cauliflower, broccoli, cabbage, kale and kohlrabi; other vegetables, such as carrot and parsley; other agriculturally grown plants, such as sugar beets, cotton, fruit trees, berry plants and grapes; and economically important tree species, such as pine, spruce, fir and aspen. The process of the present invention and the resulting bacteria may also be used to fulfill some or all of the fixed-nitrogen or other agricultural chemical requirements of leguminous plants, such as soybeans, alfalfa, clover, field beans, mung beans, peas and other pulses, as a supplement for example, to fixed nitrogen provided by species of Rhizobium associated with nodules on their roots.

Reference will now be made in detail to the presently preferred embodiments of the invention, which together with the following examples, serve to explain the principles of the invention.

The steps of the above method of the invention may be performed in any convenient order. Preferably, they are performed in the order recited with the selectable traits of the agricultural-chemical-producing bacterium screened for in step C(1) including the ability of the hybrid to produce the agricultural chemical in question, as in the case of fixed-nitrogen producing bacteria. If screening for ability to produce the agricultural chemical in question is not conducted in the first screening step, then it is preferred to conduct such screening (step C(4) prior to or concurrently with the screening in the plant host contemplated by steps C(2) and C(3). When performed in the order recited, the antibiotic resistance markers or the like constituting the selectable traits associated with the infecting bacterium need not survive subsequent screening procedures.

The stable fusion hybrid bacteria resulting from processes of the present invention are distinguished from hitherto known organisms in that they have both the ability to produce agricultural chemicals and the ability to enter into non-pathogenic endosymbiotic relationships with a plant host. The endosymbiotic relationship referred to is one in which the organism actually exists within and spreads throughout all or a portion of the plant host, without causing a pathogenic response, deriving some or all of its energy requirements from carbohydrates and other materials produced by the plant host and providing agricultural chemicals which may be used by the plant host to supplement those otherwise available.

The plant hosts with which the microorganisms of the present invention may establish endosymbiotic

EP 0 125 468 B1

relationships may include virtually all economically important crop plants. The process of the present invention has been shown to be capable of producing stable fusion hybrid bacteria having the ability to produce an agricultural chemical and of entering into endosymbiotic relationships with both monocotyledonous and dicotyledonous plant hosts. A particularly important group of plant hosts for which the products of the present invention may serve as endosymbiotic agricultural-chemical-producing bacteria are the cereals, including temperate cereals, such as wheat, triticale, barley, rye, rice and oats. Endosymbiotic agricultural-chemical-producing bacteria formed in accordance with the present invention may also be useful in supplementing the agricultural chemical, including fixed nitrogen, needs of economically important sod and forage grasses, such as brome grass, blue grass, tall fescue grass and bermuda grass. Similarly, the organisms of the present invention have a demonstrated ability to increase the yields of tropical grasses, such as sugar cane, corn, millet and sorghum. Solanaceous plants, such as potatoes, tomatoes, tobacco, eggplant and pepper are suitable plant hosts to which the organisms of the present invention may be applied, as are brassicaceous plants, such as cauliflower, broccoli, cabbage, kale and kohlrabi. Miscellaneous vegetables such as carrots and parsley; other agriculturally grown plants, such as sugar beets, cotton, fruit trees, berry plants and grapes; and economically important tree species, such as pine, spruce, fir and aspen, may also serve as plant hosts for the organisms of the present invention.

Even though plants may have symbiotic relationships with microorganisms which produce agricultural chemicals, such as leguminous plants with bacteria of the genus Rhizobium which fix atmospheric nitrogen anaerobically in root nodules, these plants are obtained in the greatest yield when provided with supplemental agricultural chemical sources through fertilisation or otherwise. Accordingly, it is envisioned that such supplemental agricultural chemicals, including supplemental fixed nitrogen, for such plants may be provided by microorganisms formed in accordance with the present invention capable of producing agricultural chemicals, including fixed nitrogen, and capable of entering into endosymbiotic relationships with these leguminous plants.

The agricultural-chemical-producing bacterium employed in the present invention capable of fixing atmospheric nitrogen aerobically and of forming stable fusion hybrids by the fusion processes of the present invention may be preferably selected from the genera Azotobacter, Azomonas, Beijerinckia, and Derxia. A particularly preferred group of organisms are those from the genus Azotobacter, such as Azotobacter vinelandii, Azotobacter paspali, Azotobacter beijerinckia and Azotobacter chroococcum. Azotobacter vinelandii ia particularly preferred because its genetics and regulation of nitrogen fixation have been extensively studied and because it has a demonstrated ability to accept and express genetic material from other bacterial genera. See G.P. Roberts, et al., "Genetics and Regulation of Nitrogen Fixation", Ann. Rev. Microbiol., 35:207-35 (1981).

Because Azotobacter and other significant aerobic nitrogen-fixing bacteria are generally gram-negative bacteria, gram-negative nitrogen-fixing bacteria are preferred for use in accordance with the present invention. It is to be understood, however, that the fusion techniques described hereinafter are applicable to both gram-positive and gram-negative bacteria, and gram positive nitrogen-fixing bacteria are envisioned as suitable for use in accordance with the processes of the present invention.

Other agricultural-chemical-producing bacteria may be employed in the present invention which are capable of producing a wide variety of antibiotics and of forming stable fusion hybrids by the fusion processes of the present invention or are capable of producing e.g. plant growth regulating compounds, fertilising chemicals other than fixed nitrogen, fragrances, sensory enhancing chemicals or antifeeding agents. Indeed, it is contemplated that any chemical or chemical effect produced by bacteria as herein defined which would be of value as an agricultural chemical and which is produced by organisms which form stable fusion hybrids with infecting bacteria as herein defined and the processes described herein may be used in accordance with the present invention. Selection of such agricultural-chemical-producing bacteria will be within the capabilities of those skilled in the art in the light of the teachings and illustrations contained herein.

A table of exemplary agricultural-chemical-producing bacteria contemplated as useful in accordance with the present invention and the agricultural chemical or chemical effects they produce appear in Table I.

8

| Group | Genus | Species | Chemical/Application |
|---|---|---|---|
| **Antibiotics** | | | |
| Antifungal Agents | Streptomyces | cacaoi var. asoensis | produces polyoxins which prevent rice sheath blight |
| | | griseus | produces cycloheximide which prevents onion downy mildew |
| | | griseochromagenes | produces blasticidin S which prevents rice blast |
| | | hygroscopicus var. limoneus | produces validamycin A which prevents rice sheat blight |
| | | kasugaensis | produces kasugamycin which prevents rice blast |
| | | kitazawaensis | produces ezomycin which prevents stem rot of kidney beans |
| | | Tendae Tu 901 | produces nikkomycin which is active against various fungi |
| | Pseudomonas | fluorescens strain B10 | causes iron to become unavailable to other microorganisms such as fungi |

Table I.  Agricultural-Chemical-Producing Bacteria

EP 0 125 468 B1

| Group | Genus | Species | Chemical/Application |
|---|---|---|---|
| | Streptoverticillium | rimofaciens | produces mildiomycin which is active against various powdery mildews |
| Antibacterial Agents | Streptomyces | chibaensis | produces cellocidin which prevents rice bacterial leaf blight |
| | | griseus | produces streptomycin which prevents various bacterial diseases |
| | | lavendulae No. 6600 Gc-1 | produces laurusin which prevents bacterial leaf blight of rice plants |
| | | spheroides | produces novobiocin which prevents tomato canker |
| | | venezuelae | produces chloramphenicol which prevents bacterial leaf blight of rice plants |
| | | viridifaciens | produces tetracycline which prevents various bacterial diseases |
| | Agrobacterium | radiobacterial strain | produces agrocin - 84 |

EP 0 125 468 B1

| Group | Genus | Species | Chemical/Application |
|---|---|---|---|
| | Nocardia | sp. | produces myomycin |
| Antiviral Agents | Streptomyces | hygroscopicus var. aabomyceticus | produces aabomycin A which inhibits TMV multiplication in tobacco tissue |
| | | lavendulae No. 6600 Gc-1 | produces laurusin which inhibits TMV multiplication in tobacco tissue |
| | | miharuensis | produces miharamycin which is effective in controlling TMV, CMV, PVX, and RSV |
| Insecticides | Bacillus | cereus | affects hymenoptera, lepidoptera, coleoptera |
| | | euloomarahae | affects coleopteran larvae, especially Japanese beetle |
| | | fribougensis | affects coleopteran larvae, especially Japanese beetle |
| | | lentimorbus | affects coleopteran larvae, especially Japanese beetle |
| | | moritai | affects seedcorn maggot |
| | | popilliae | affects coleopteran larvae, especially Japanese beetle |

EP 0 125 468 B1

| Group | Genus | Species | Chemical/Application |
|---|---|---|---|
| | | thuringiensis<br>var. alesti<br>dendrolimus<br>entomocidus<br>kurstaki<br>sotto | affect numerous lepidopteran pest larvae |
| | Clostridium | bravidaciens | affects tent caterpillar |
| | | malacusomae | affects tent caterpillar |
| | Streptomyces | strain B-41-146 | produces milbemycin |
| Nematocide | Bacillus | penetrans | effective against root-knot nematodes |
| Miticide | Streptomyces | aureus S-3466 | produces tetranactin |
| Herbicides | Streptomyces | griseus | produces cycloheximide |
| | | saganoensis | produces herbicidin A, B |
| | | sp. | produces bialaphos |
| | | sp. | produce anisymycin |
| | Pseudomonas | syringae pv.<br>atropurpurea | produces coronatine which causes chocolate spot disease on Italian ryegrass |

| Group | Genus | Species | Chemical/Application |
|---|---|---|---|
| Fertilizers | | | |
| Nitrogen-fixing | Azotobacter | beijerinckia, enroococcum, paspali, vinelandii | fix atmospheric nitrogen |
| | Azomonas | | fix atmospheric nitrogen |
| | Beijerinckia | | fix atmospheric nitrogen |
| | Derxia | | fix atmospheric nitrogen |
| Phosphate Solubilizing | Bacillus | circulans, flourescens, megaterium, mesentericus, mycoides, polymyxa, pulvitaciens subtilis | solubilize bound phosphate |
| | Pseudomonas | calcis, liquifaciens, putida, rathonia, striata | solubilize bound phosphates |

EP 0 125 468 B1

| Group | Genus | Species | Chemical/Application |
|---|---|---|---|
| Plant Growth Regulators | Azotobacter | sp. | produce various vitamins auxins, cytokinins, gibberellin-like substance and other plant hormones. |
| | Agrobacterium | sp. | |
| | Pseudomonas | sp. | |
| | Bacillus | megaterium, subtilis | |

The infecting bacterium capable of infecting the plant host employed in the present invention may be any of a wide variety of bacterial species which infect the plant host under consideration and which are capable of forming stable fusion hybrids in accordance with the fusion techniques discussed hereinafter. Preferably, the infecting bacterium should have a known method of interaction with the plant host during the initial phase of infection. The infecting bacteria may be either a pathogen, including latent pathogens, or an endosymbiotic species. Exemplary pathogens include species of the genera Agrobacterium and Erwinia. Exemplary endosymbiotic species include species of Azospirillum, which are known to live in the roots of tropical grasses, such as sugar cane, and temperate grasses, such as wheat, without causing manifestations of disease. In the case of pathogens, it is preferred that the pathogen create a visible manifestation of the disease associated with it.

Most preferably, the infecting bacterium is a strain which is specific, or nearly specific, to the particular crop plant host under consideration in order to limit the potential for spread of the fusion hybrids of the present invention to plants other than those with which they are intended to enter an endosymbiotic relationship. Many plant infecting bacteria and their methods of interaction with plant hosts are described in the scientific literature. Particular reference in this regard is made to M.S. Mount et al., Phytopathogenic Prokaryotes, Volumes I and II (1982).

Many pathogenic organisms are known to form organism strains essentially undifferentiable from each other except for their preference for particular plant hosts. Such host-specific pathogen strains are known as pathovars for the plant host involved. It is envisioned that the present invention will be particularly useful with pathovars for the particular plant host under consideration, including pathovars of the genera Agrobacterium, particularly Agrobacterium tumefaciens; Erwinia, particularly Erwinia stewartii and Erwinia carotovora; Pseudomonas, particularly Pseudomonas solanacearum and Pseudomonas syringae; Xanthomonas, particularly Xanthomonas campestris; and similar bacteria.

Particularly preferred non-pathogenic endosymbionts include species of the genus Azospirillum, particularly Azospirillum lipoferum and Azospirillum brasilense. Infecting bacteria envisioned as suitable for use in accordance with the present invention include those listed in Table II.

15

Table II

| Plant-Infecting Bacteria | | |
|---|---|---|
| Group | Genus | Species |
| Gram-negative aerobic rods and cocci | Pseudomonas<br><br><br>Xanthomonas<br>Agrobacterium | agarici, andropognis, avenae, caricapapayae, caryophylli, cichorii, cissicola, gladioli, glumae, marginalis, pseudoalcaligenes subsp. citrulli, rubrilineans, rubrisubalbicans, solanacearum, syringae, tolaasii, viridiflava<br>albilineans, ampelina, axonopodis, campestris, fragariae<br>rhizogenes, rubi, tumefaciens |
| Gram-negative facultatively anaerobic rods<br><br><br><br>Gram-negative<br>Gram-positive Actinomycetes and related organisms | Erwinia<br><br><br><br>Azospirillum<br>Coryne- bacterium insidiosum, | amylovora, ananas, cancerogena, carnegieana, carotovora subsp. atroseptica, carotovora subsp. carotovora, chrysanthemi ,cypripedii, dissolvens,herbicola, mallotivora, milletiae, nigrifluens, nimipressuralis, quercina, rhapontici, rubrifaciens, salicis, stewartii, tracheiphila, uredovora<br>lipoferum, brasilense<br>betae, beticola, fascians, flaccumfaciens, ilicis, michiganense, nebraskense, oortii, poinsettiae, sepedonicum |

Pathovars of Agrobacterium tumefaciens are particularly preferred for formation of stable fusion hybrids capable of producing agricultural chemicals and of entering into endosymbiotic relationships with dicotyledonous plant hosts. Agrobacterium tumefaciens has a well identified interaction with the plant during the intitial phases of infection in that it binds to plant cell tissue. The ability of Agrobacterium tumefaciens to bind to plant cell tissue in vitro has been demonstrated and has been shown to parallel the host range for tumor formation by Agrobacterium tumefaciens in vivo. See A.G. Matthysse, et al., "Plant Cell Range for Attachment of Agrobacterium tumefaciens to Tissue Culture Cells," Physiological Plant Pathology, 21: 381-387 (1982); A.G. Matthysse, et al., "Binding of Agrobacterium tumefaciens to Carrot Protoplasts," Physiological Plant Pathology, 20: 27-33 (1982).

Agrobacterium tumefaciens is the preferred infecting bacterium for use in performing the invention with solanaceious plants, such as potatoes, tomatoes, tobacco, eggplant and pepper; brassicaceous plants, such as cauliflower, broccoli, cabbage, kale and kohlrabi; vegetables, such as carrot and parsley and other agriculturally grown plants, such as sugar beets, cotton, fruit trees, berry plants and grapes. Other infecting bacteria suitable for use in accordance with the present invention include Erwinia carotovora, Pseudomonas solanacearum, Pseudomonas syringae and Xanthomonas campestris.

Infecting bacteria such as Pseudomonas syringae and Xanthomonas campestris are envisioned as being particularly useful in performing the invention with monocotyledonous cereal crops, such as wheat, barley, rye, rice and oats, and also with grasses, such as brome grass, blue grass, tall fescue grass and bermuda grass.

Strains of Erwinia stewartii are particularly preferred as the infecting bacteria with monocotyledonous plants, such as tropical grasses, such as sugar cane, corn, millet and sorghum, and small grains such as rice. Strains of Pseudomonas syringae and Xanthomonas campestris are also envisioned as useful in these applications.

The above-identified strains of Azospirillum are envisioned as being useful in monocotyledonous plants, including tropical and temperate grasses, such as sugar cane and wheat, among others.

It is to be understood that the selection of a particular plant-infecting bacterium and a particular agricultural-chemical-producing bacterium for use in accordance with the present invention will be within the capabilities of one having ordinary skill in the art through the exercise of routine experimentation in light of the teachings contained herein and inferences logically to be drawn from the disclosure of the present invention. The characteristics of bacteria contemplated as useful as either the agricultural-chemical-producing bacterium or the infecting bacterium in processes of the present invention may be determined by routine experimentation or by reference to standard compendia, such as Bergey's Manual of Determinative Bacteriology.

For use in the present invention, mutants of the desired infecting bacterium should be selected which have one or more selectable traits in addition to the ability to infect the plant host under consideration. Specifically, mutants of the infecting bacterium should be selected which have traits which are selectable in vitro. Such traits include antibiotic resistance, or the need for specific nutritional supplementation (auxotrophism) or combinations thereof, which will allow rapid in vitro screening of the hybrid fusion products to identify those containing genetic material from the mutant infecting bacterium.

Antibiotic resistance is the preferred selectable trait, and it is particularly preferred that the selected infecting-bacterium mutant have resistance to at least two antibiotics. Dual antibiotic resistance, or redundancy in other selectable traits, ensures that the initial screening of the fusion hybrids will identify only those having genetic material from both the agricultural-chemical-producing bacterium and the selected infecting-bacterium mutant and reduces the chances of survival or formation of mutant antibiotic resistance strains of the agricultural-chemical-producing bacterium. For example, in formation of agricultural-chemical-producing hybrids capable of producing fixed nitrogen and of entering into endosymbiotic relationships with monocotyledonous plants, strains of Erwinia stewartii which have resistance to both streptomycin and tetracycline are preferred. In the formation of hybrids capable of producing fixed nitrogen and of entering into endosymbiotic relationships with dicotyledonous plants, strains of Agrobacterium tumefaciens which are both streptomycin resistant and kanamycin resistant are preferred. Such mutant strains of plant infecting bacteria are available from microorganism depositories such as the American Type Culture Collection in Rockville, Maryland, or may be generated from the wild by techniques well known in the art.

Where hybrids of the agricultural chemical producing bacteria are not to be screened initially for the ability to produce the agricultural chemical in question, the agricultural-chemical-producing bacterium used in the fusion step may be a mutant with one or more additional selectable traits, preferably in vitro selectable traits, such as those mentioned above with respect to the infecting bacterium. Thus, for example, if the agricultural chemical of interest is e.g. an insecticide or hematocide, the agricultural-chemical-producing bacterium may be a mutant which has selectable antibiotic resistance or sensitivity or a selectable need for specific nutritional supplementation. Obviously, these traits should not be identical to the selectable traits of the infecting bacterium since the initial screen would otherwise be unable to eliminate non-hybrid organisms. While the presence in the hybrids of such additional selectable traits from the agricultural-chemical-producing bacterium does not guarantee the presence of agricultural-chemical-producing capability in the products of the initial screening procedure, it sufficiently enriches the population with true hybrid organisms to the point where there is a reasonable predictability of success in identifying those hybrid organisms with the agricultural-chemical-producing capability in subsequent screening.

The fusion hybrids of the present invention are formed by protoplast or spheroplast fusion techniques following the outline of techniques generally employed in the prior art. Known protoplast and spheroplast fusion techniques are described in D.A. Hopwood, "Genetic Studies With Bacterial Protoplasts", Ann. Rev. Microbio., 35: 237-72 (1981), and in R.L. Weiss, J. Bacteriol., 128: 668-70 (1976).

Selection of a technique by which the fusion hybrids of the present invention may be formed will generally be within the capabilities of one of ordinary skill in the art based on the above-referenced scientific literature and the insights gained through study of the protoplast fusion examples hereinafter set forth.

In general, the fusion procedure involves the removal of the cell wall from both the agricultural-chemical-producing bacteria and the infecting bacteria, fusion of the infecting bacteria and agricultural-chemical-producing bacteria cells in a fusion-inducing medium, such as polyethylene glycol, and regeneration of the cell wall about the fusion hybrids containing genetic material from both the infecting bacterium and the agricultural-chemical-producing bacterium. Fusion and regeneration of the cell wall are conducted at low temperatures so that rates of expressible genetic recombination are favored in relation to rates of enzymatic destruction of genetic material in the newly formed hybrids.

Following initial formation of fusion hybrids, the genetic make-up of the bacterial population is relatively unstable for a period of two or three days, during which much genetic recombination apparently occurs. During this time period, those stable fusion hybrids capable both of manifesting the one or more selectable traits associated with the agricultural-chemical-producing bacteria and of manifesting the one or more selectable traits associated with the infecting bacterium are selected. It is particularly preferred that the agricultural-chemical producing trait also be selected during this step. For example, fusion products of Azotobacter vinelandii and Erwinia stewartii formed from strains of Erwinia stewartii which are streptomycin and tetracycline resistant are grown in a nitrogen-free medium containing both streptomycin and tetracycline. Thus, only those organisms containing both the genetic material associated with nitrogen fixation from Azotobacter vinelandii and the genetic material associated with streptomycin and tetracycline resistance from Erwinia stewartii will survive the two to three day incubation period.

The surviving fusion hybrids manifesting both the selectable straits associated with the agriculturalchemical-producing bacterium and the selectable traits associated with the infecting bacterium may then be screened for their ability to interact with plant tissue in the manner in which the infecting bacterium interacts with plant tissue during the initial phase of infection of the plant host. As above noted, the initial phase of infection associated with Agrobacterium tumefaciens is binding to plant tissue cells. The ability of the hybrids to bind to plant tissue cells may be determined in vitro by techniques hitherto described in the above-cited literature and by techniques described in the following examples.

Other infecting bacteria , such as Erwinia stewartii, initially interact with plant tissue following infection by spreading throughout the vascular system of the plant without being detected and destroyed by the plant's disease-response system. This character is assumed in the literature to be due to an extracellular polysaccharide produced by the infecting bacterium which permits it to elude the plant's normal defensive reaction. The ability of the fusion hybrids to spread throughout the vascular system of the plant in this manner may be determined in any convenient manner. One preferred screening technique involves the infection of seedlings of the host plant at a particular site in the plant. After a period of time, e.g., four days, the plant may then be dissected into a plurality of transverse sections displaced along the longitudinal axis of the plant. Bacterial cultures may be regenerated from the bacteria contained in each section. The bacteria -containing sections farthest removed from the situs of initial infection will contain those fusion hybrids best able to disperse throughout the vascular system of the plant, thereby allowing selection of hybrids having this ability.

It is to be understood that other techniques for screening for this initial interaction with plant tissue may be envisioned by those skilled in the art in light of the teachings contained herein. It is to be further understood that other mechanisms of initial interaction between particular infecting bacteria and particular plant hosts may be quantified and screened for in ways which will be obvious to those skilled in the art in light of the teachings contained herein and known interactions between infecting bacteria and their plant hosts.

The ability to spread throughout the plant's vascular system is a desirable property even for fusion hybrids formed from infecting bacteria whose initial interaction with plant cell tissue is by some other mechanism, e.g., cell binding. Accordingly, it is preferred to screen further those fusion hybrids found to possess both the ability to produce agricultural chemical and the ability to interact with plant cell tissue in the manner in which the infecting bacterium interacts with the plant host during the initial phases of infection to select a subgroup of hybrids for further screening according to the present invention which are also capable of dispersing quickly throughout the vascular system of the plant.

Moreover, in practicing the present invention, it is preferred that the fusion hybrids found to have both the ability to produce agricultural chemicals and the ability to interact with plant cell tissue in the manner in which the infecting bacterium interacts with plant cell tissue during the initial phases of infection be screened for these capabilities two or more times. Such repeated screening tends to assure stability of the fusion hybrid strain and also allows selection for further screening of a manageable number of fusion hybrids having the greatest capabilities in these two areas.

The stable fusion hybrids manifesting both the selectable traits of agricultural-chemical-producing bacterium and plant-tissue-interaction capabilities, as above described, may then be grown as individual colonies, in the case of the agricultural-chemical-producing bacterium that fix nitrogen, preferably on nitrogen-free media. Each of the resultant cultures may then be applied in an appropriate manner, e.g., by injection to seedlings of the plant host in question. After an appropriate incubation period, e.g., two or three weeks, those fusion hybrids which do not create any visible manifestation of a disease in the host plant seedlings may be selected for further screening. Experience has shown that screening of 25 to 30 of the best fusion hybrids is normally sufficient to provide 5 to 7 fusion hybrids which do not manifest visible disease symptoms, such as any associated with the original infecting bacterium, in the host plant in question.

Preferably, those stable fusion hybrids which manifest the widest area of spread throughout the vascular system of the plant, the clearest freedom from symptoms of disease in the host plant, and, in the case of agricultural-chemical-producing bacteria that fix nitrogen, the most vigorous growth in nitrogen-free media, are selected for further screening.

If not conducted in the initial screening of hybrid organisms, the hybrid organisms must, sooner or later, be screened directly for the ability to produce the agricultural chemical of interest. Preferably, the screening should be conducted at the earliest practical point in the process, which will be dictated by the number of hybrid organisms surviving at any particular stage of the process and the power of the technique used to screen for production of the agricultural chemical of interest to identify agricultural-chemical-producing hybrids out of a mixed inoculum.

In general, these screening prodecures will fall into one of four categories. These are (1) survival screens, where the agricultural chemical being produced is essential to survival of the hybrid and is not provided by the growth media; (2) analytical chemistry screens for the presence of the agricultural chemical being produced; (3) biological screens for the manifestations of biological activity associated with the agricultural chemical or chemicals being produced; and (4) immunoassay screens whereby organisms or groups of organisms producing a particular agricultural chemical are identified by interaction with an antibody which is specific to the chemical in question. The suitability of any particular screening technique or combination of techniques will be dictated by the agricultural chemical in question. Thus, survival screens are particularly useful in identifying hybrid bacteria capable of producing their own fixed nitrogen by survival on nitrogen-deficient media. Biological screens are particularly useful in identifiying hybrid bacteria capable of producing anti-bacterial or anti-fungal agents. A particularly preferred screening technique of this sort involves layering a culture of organisms susceptible to the antibiotic produced by the hybrid bacteria over a mixed culture of hybrids and identifying and removing for further screening hybrids or groups of hybrids producing that antibiotic from those areas where the susceptible organism cannot grow. Immunoassay techniques are particularly appropriate where the agricultural chemical of interest is a large molecule, such as a polypeptide. In any event, biological screens for the known effects of the agricultural chemical in question can be conducted using discrete cultures of hybrid organisms once the number of such cultures has been reduced to a manageable level, e.g., $10^2$ - $10^3$ cultures, by preliminary screens for other selectable traits associated with the agricultural-chemical-producing bacterium. Thus, hybrids producing insecticidal compounds, for example, could be identified by the inability of susceptible insects to survive on a culture of the hybrid. Such screening has the disadvantage of requiring screening of a larger number of colonies and requiring a protracted period of time to manifest results but is nonetheless capable of identifying hybrids with the ability to produce the agricultural chemical of interest.

It is to be understood that the screening for a particular agricultural-chemical-producing trait for use in

19

accordance with the present invention will be within the capabilities of one having ordinary skill in the art through the exercise of routine experimentation in light of the teachings contained herein and inferences logically to be drawn from the disclosure of the present invention. The techniques contemplated as useful in screening for ability to produce the agricultural chemical may be determined by reference to standard compendia.

In order to determine which of the final group of fusion hybrids is capable of entering into endosymbiotic relationships with the host plant whereby agricultural chemicals produced by the hybrid may be utilized by the plant as a supplement to agricultural chemicals otherwise available, it is normally necessary to conduct preliminary yield gain screening or the like. For nitrogen-fixing bacteria , for example, this is accomplished by growing the host plant in an environment deficient in the agricultural chemical, e.g., fixed nitrogen, and comparing the dry weight after an appropriate period of growth, e.g., six weeks, with the dry weight of similar plants in similar environment but which had been infected, as by injection, with the stable fusion hybrid bacteria selected by the foregoing method. In the case of agricultural-chemical-producing bacteria that fix nitrogen, experience has shown that testing of 5 to 7 of the best fusion hybrids will result in identification of 3 or 4 stable fusion hybrids capable of creating statistically significant yield gains in the host plant growing in nitrogen deficient soil due, it is believed, to the ability of the stable fusion hybrids to fix atmospheric nitrogen aerobically and to enter into endosymbiotic relationships with the host plant.

It is preferred in conducting the process of the present invention, although not necessary, that the infecting bacterium and the agricultural-chemical-producing bacterium employed exhibit the same response to gram stain. Moreover, it is envisioned that the endosymbiotic agricultural-chemical-producing bacteria formed in accordance with the present invention may be further modified by natural or artificial genetic techniques to improve their performance as sources of agricultural chemicals for the plant host. Specifically, the bacteria may be modified to reduce their resistance to cold temperatures, thereby preventing unintended proliferation of the hybrid bacteria from year to year. Similarly, the endosymbiotic hybrid bacteria formed in accordance with the present invention may be modified to enhance their stability to drought, disease or other physiological stress. In this regard, the stability of the hybrid under stress conditions, and the ability to minimize the likelihood of spontaneous or forced reversion to a pathogenic state, is desirable in a commercial microbiological product. Ideally, the bacteria formed in accordance with the present invention should be modified so that they cannot grow outside the plant host, as by the selection of mutants requiring nutritional supplementation specifically or nearly specifically provided by the plant host in question. Techniques for genetic manipulation and mutant selection for strains of, for example, Azotobacter are well known in the art and are envisioned as being suitable for effecting the above-described and similar genetic manipulations of the stable fusion hybrids formed in accordance with the present invention.

The stable fusion hybrid bacteria having the ability to produce agricultural chemicals and of entering into an endosymbiotic relationship with a plant host formed in accordance with the present invention may be used to prepare agricultural products in many ways. For example, bacteria formed in accordance with the present invention may be injected into seedlings or used to form coated seed of the plant host involved by association of the bacteria with a biodegradable nutrient carrier material which is coated on the seed. Similarly, bacteria formed in accordance with the present invention may be used to form infected seed products by application of the bacteria directly to the seed of host plants. Alternatively, an agricultural soil drench may be prepared by mixing bacteria formed in accordance with the present invention with water and other appropriate drench constituents for application to the leaves, stem and roots of growing host plants and surrounding soil by spraying or the like.

It is to be understood that application of the teachings of the present invention to a specific problem or environment will be within the capabilities of one having ordinary skill in the art in the light of the teachings contained herein. Examples of processes within the scope of the present invention and the bacterial products resulting therefrom appear in the following examples.

Example 1

Preparation of a Fusion Hybrid Bacterium from Azotobacter vinelandii and Erwinia stewartii Capable of Fixing Atmospheric Nitrogen Aerobically and of Entering Into an Endosymbiotic Relationship with Corn

Approximately $10^8$ bacteria of the species Azotobacter vinelandii known to be capable of fixing nitrogen aerobically were selected. Approximately $10^8$ bacteria of a strain of Erwinia stewartii mutants known to be resistant to both streptomycin and tetracycline were also selected. Fusion hybrids of these two types of bacteria were formed by a variant of the procedure of Weiss referred to above.

The bacteria growing in mid-log phase were treated with lysozyme at a concentration of 100 micrograms per milliliter and with DNA-ase at a concentration of 5 micrograms per milliliter in media containing EDTA at a concentration of 1 millimolar and magnesium chloride at a concentration of 5 millimolar, having a pH of 7 and with 12% added sucrose. After 10 minutes, protoplasts and spheroplasts of the original bacteria were formed. The temperature was then lowered to 0° C and the protoplast-forming solution was washed out. The resulting protoplasts were placed in 12% sucrose solution at 0° C. The solution was then supplemented to 40% polyethylene glycol of molecular weight about 6,000 in order to induce protoplast and spheroplast fusion for a period of 10 to 15 minutes. The fused bacteria were spun down in a centrifuge and resuspended in 12% sucrose plus a complete growth medium for 4 hours at 0° C. The temperature was then raised to 30° C and was held there for 2 hours, after which the hybrid fusion products were transferred to complete growth medium containing no additional sucrose for 2 hours.

The initial selection procedure was then conducted at a temperature of about 25-30° C.

The hybrid fusion products were transferred and grown on Burke's nitrogen-free medium as described in Carlson, et al., "Forced Association Between Higher Plant and Bacterial Cells in Vitro" Nature, Vol. 22, No. 5482, p. 393-395 (1974). The medium was nitrogen-free and contained added streptomycin at a concentration of 100 micrograms per milliliter and tetracycline at a concentration of 50 micrograms per milliliter. Only those fusion products manifesting both the ability to fix atmospheric nitrogen aerobically and manifesting the streptomycin and tetracycline resistance associated with the Erwinia Stewartii strain originally introduced survived on this medium after a growth period of about 3 days.

Samples of the mixed fusion hybrids from this culture were used to infect corn seedlings approximately 3 to 4 feet in height by injection between the first and second node of the stalk tissue. After 4 days the corn plants were dissected into transverse sections to recover bacteria that moved to the anterior (top) of the plant, since Erwinia Stewartii interacts with plant tissue during the initial phase of infection by spreading throughout the vascular system of the plant without being recognized or destroyed by the plant's disease-response system. Those fusion hybrids with the greatest facility to spread throughout the vascular system of the corn plant were generally found between the fifth and sixth node.

The fusion hybrid bacteria found between the fifth and sixth node of the corn plant were cultured for 3 days on Burke's nitrogen-free medium as described by Carlson, et al., supra.

After 3 days, a mixed inoculant from this nitrogen-free culture was again used to infect corn seedlings approximately 3 to 4 feet in height by injection between the first and second node of the stalk tissue. After about 3 days the plant was transversely dissected as above described to recover bacteria that moved to the anterior of the plant. Again, those fusion hybrids with the greatest facility to spread throughout the vascular system of the corn plant were generally found between the fifth and sixth node.

The fusion hybrid bacteria recovered from this portion of the corn plant were then grown as separate colonies on Burke's nitrogen-free medium. Twenty-five of the most vigorous colonies were selected, and each was used to infect one of 25 corn plants by injection. After 3 weeks, those colonies which did not result in any visible manifestation of disease in the infected corn plants were selected. From among these, seven solonies which manifested the most vigorous growth on nitrogen-free media, the widest area of spread throughout the plant and clearest freedom from symptoms of disease were selected for preliminary yield testing and were identified as Fusion #1 through Fusion #7.

In the preliminary yield test, flats (30cm x 46cm) containing 15cm of washed sterile sand over 10cm of Perlite were sown with 24 corn seeds at a uniform spacing. Flats were maintained in the greenhouse (at approximately 22-27° C) and were watered twice daily with deionized water. Starting at 10 days (so as to exhaust the seed nitrogen) the seedlings were watered three times a week with a nutrient solution. One liter of solution was applied and allowed to drain through. The solution was a modified Long Ashton mix that contained nitrate ion at a concentration of 6 micromolar as its sole nitrogen.

The Long Ashton mix was also modified to contain nitrate ion at concentrations of 6 millimolar and 0 and used in comparative runs A and C below, respectively. Experiments were run for six weeks, at which time the aerial portions of the plants were harvested and dried for one week at 95° C. Dry weight data was then collected. For flats containing infected plants, the bacterial strains were introduced by injection at 10 days (at the same time as fertilization was initiated). Each analysis was done in triplicate (three flats of 24 plants per treatment) and the data were subjected to statistical analysis. Seven different bacterial fusions were selected by multiple screens between Erwinia stewartii and Azotobacter vinelandii (Fusion #1 through 7) as above described and used in this work. Actual dry weight yield data per flat (average of the three flats) is as follows:

| Inoculation | NO$_3$ Level | Average Flat Dry Weight (grams) | Percentage Increase Over Control |
|---|---|---|---|
| A. None | 6 millimolar | 116.8 | - |
| B. None (Control value) | 6 micromolar | 34.8 | - |
| C. None | None | 19.2 | - |
| Fusion #1 | 6 micromolar | 44.8 | 29 |
| Fusion #2 | 6 micromolar | 39.6 | 14 |
| Fusion #3 | 6 micromolar | 72.4 | 108 |
| Fusion #4 | 6 micromolar | 33.2 | -5 |
| Fusion #5 | 6 micromolar | 54.8 | 57 |
| Fusion #6 | 6 micromolar | 25.2 | -28 |
| Fusion #7 | 6 micromolar | 56.8 | 63 |

Results with Fusion numbers 1, 3, 5, and 7 are statistically significant.

Uninoculated comparative run A contains the optimum level of nitrate in Long Ashton nutrient solution and is indicative of results to be expected with corn growing in well nitrogen-fertilized soil. Comparative run C, to which no nitrate was added, shows the yields which may be expected based only on residual nitrate contained in the seed of the corn. Comparative run B, which represents the control value containing nitrogen at levels of about 6 micromolar, is indicative of growth to be expected in poorly nitrogen-fertilized soil. The reduction in dry weight associated with fusion product number 6 is apparently due to non-visible pathogenic effects, even though none of the plants manifested visual symptoms of disease. The results of fusion product number 4 are not statistically significant. The results of fusion products 1, 3, 5, and 7 show that the process of the present invention was able to produce at least four stable fusion hybrid products capable of fixing atmospheric nitrogen aerobically and capable of entering into endosymbiotic relationships with corn whereby corn growing under nitrogen stressed conditions in conjunction with the fusion products of the present invention produced yields of from 29 to 108% greater than a control without the bacteria formed in accordance with the present invention.

Example 2

Preparation of a Fusion Hybrid Bacterium from Azotobacter vinelandii and Agrobacterium tumefaciens Capable of Fixing Atmospheric Nitrogen Aerobically and of Entering Into an Endosymbiotic Relationship with Sugar Beets

Approximately 10$^8$ bacteria of the species Azotobacter vinelandii were selected. Approximately 10$^8$ bacteria of a strain of Agrobacterium tumefaciens mutants which are resistant to both streptomycin and kanamycin were selected.

Protoplast fusion was effected between these bacteria as in Example 1. The fused protoplasts were transferred to Burke's nitrogen-free medium as described in Example 1 supplemented with streptomycin at a concentration of 100 micrograms per milliliter and kanamycin at a concentration of 50 micrograms per milliliter. After 3 days, only those stable fusion hybrids capable of both fixing atmospheric nitrogen aerobically and possessing the genetic material from the pathogenic bacteria associated with streptomycin and kanamycin resistance survived.

A mixed inoculum from this culture was introduced into a suspension culture of carrot tissue cells in the manner described by A.G. Matthysse, et al, Physiological Plant Pathology, 20, 27-33 and 21, 381-387 (1982). After about 3 hours, the carrot cells and those fusion hybrids capable of binding to carrot cells in a manner similar to the initial interaction of Agrobacterium tumefaciens with plant tissue cells during the initial phases of infection were separated from unbound bacteria by washing 3 times in sterile, distilled water. The carrot cells with adhering hybrid bacteria were macerated and washed again. The resulting suspension was spun down in a centrifuge and the resulting pellet, consisting of cell walls and adhering hybrid bacteria, was placed on Burek's nitrogen-free medium for 3 days.

The cell-binding method as above-described was repeated and the fusion hybrids thus selected were grown as separate colonies on nitrogen-free media. After 3 days of growth, 25 of the most vigorous colonies were selected.

Each colony was used to infect one of 25 sugar beet seedlings 8 to 10 inches high by insertion of a pin, which had been dipped into the colony, into the area of the hypocotyle between the root and the plant. Those hybrid colonies which did not form a gall or tumor in the sugar beet seedling after 3 weeks were selected for further screening.

The roots of each infected sugar beet plant which did not manifest any visible symptoms of disease after 3 weeks were then dissected into a number of transverse sections. The distance down the root of the plant to which the hybrid bacteria had spread was noted. Five of the 25 stable fusion hybrids were selected for preliminary yield testing based upon the vigorousness of their growth on nitrogen-free media, the absence of visible manifestations of disease associated with their injection into the plant and the facility with which they were able to spread throughout the plant tissue. These five fusion hybrids, designated Fusion #1 through Fusion #5 were subjected to preliminary yield testing as follows.

Flats (30cm x 46cm) containing 15cm of washed sterile sand over 10cm of Perlite were plated with 24 germinated sugar beet seeds at a uniform spacing. Flats were maintained in the greenhouse (at approximately 22-27° C) and were watered twice daily with deionized water. Starting at 10 days (so as to exhaust the seed nitrogen) the seedlings were watered three times a week with a nutrient solution. One Liter of solution was applied and allowed to drain through. The solution was a modified Long Ashton mix that contained nitrate ion at a concentration of 6 micromolar as its sole nitrogen. The Long Ashton mix was also modified to contain nitrate ion at concentrations of 6 millimolar and 0 and used in comparative runs A and C below, respectively.

Experiments were run for six weeks, at which time the plants were harvested and dried for one week at 95° C. Dry weight data was then collected. For flats containing infected plants, the bacterial strains were introduced by injection at 10 days (at the same time as fertilization was initiated). Each analysis was done in triplicate (three flats of 24 plants per treatment) and the data were subjected to statistical analysis. Five different bacterial fusions were selected by multiple screens between Agrobacterium tumefaciens and Azotobacter Vinelandii as above described and used in this work. Actual dry weight yield data per flat (average of the three flats is as follows:

| Inoculation | NO$_3$ Level | Average flat Dry Weight (grams) | Percentage Increase Over Control |
|---|---|---|---|
| A. None | 6 millimolar | 44.7 | - |
| B. None (control value) | 6 micromolar | 8.8 | - |
| C. None | None | 5.2 | - |
| Fusion #1 | 6 micromolar | 8.0 | -9 |
| Fusion #2 | 6 micromolar | 12.4 | 41 |
| Fusion #3 | 6 micromolar | 6.9 | -22 |
| Fusion #4 | 6 micromolar | 24.6 | 180 |
| Fusion #5 | 6 micromolar | 14.9 | 69 |

Results with Fusion numbers 2, 3, 4 and 5 are statistically significant.

Uninoculated comparative runs A, B, and C have the same significance as uninoculated comparative runs A, B, and C in Example 1 above. Fusion product No. 3 appeared to result in non-visible pathogenic effects, even though no visible symptoms of disease were observed on any of the plants. The prelminary yield data confirms that the process of the present invention was capable of producing at least three stable hybrid bacteria (Fusion #'s 2, 4 and 5) capable of fixing atmospheric nitrogen aerobically and of entering into an endosymbiotic relationship with sugar beets whereby sugar beets growing in nitrogen stressed conditions generated yields 41 to 180% in excess of those obtained with uninoculated sugar beets.

Example 3

Example 2 was repeated with the exception that the fusion products were applied to sugar beets receiving nitrogen in the form of nitrate at a concentration of 6 millimolar -- that associated with optimal nitrogen ferilization. No significant yield gain above that attained by uninoculated comparative run A was observed in the inoculated sugar beets. These results confirm that the yield gains associated with the fusion products in Example 2 were due to an endosymbiotic relationship between the fusion products and the plant whereby fixed nitrogen was provided by the fusion products and used by the plant host. Had the yield gains reported in Example 2 been the result of growth regulators or hormones provided by the fusion products, similar gains should have been observed in Example 3.

Further evidence of an endosymbiotic association with the host is provided by light microscopy using gram stain and from reisolation of the bacteria (with the expression of the maintained antibiotic resistance markers) from infected plants. Moreover, the hybrid bacteria can be found throughout the plant. Their location is not limited to the site of infection. The number of hybrid bacteria in plant tissue was approximately $10^5$ per gram (dry weight) in a corn plant growing under low nitrate conditions.

It is to be understood that, with appropriate changes in the screening procedure, hybrids capable of fixing nitrogen anaerobically, as is done in Rhizobium, or in the presence of very small amounts of oxygen, as is done by microaerophilic bacteria, can be prepared in accordance with the present invention. Specifically, oxygen must be minimized or removed in the nitrogen-free growth medium during screening for nitrogen-fixing ability.

If such anaerobic nitrogen-fixing hybrids are to enter into endosymbiotic relationships with plant hosts, the hybrid must also contain genetic material, such as that contained in species of Rhizobium, which will cause the plant host to create an oxygen free or low oxygen environment not normally found in crop plants. For this reason utilization of aerobic nitrogen-fixing bacteria is preferred.

Some of the fusion products of the present invention have also been observed to form spores. Selection of endosymbiotic bacteria formed in accordance with the present invention which form spores facilitates application and survival of the bacteria when applied to plant hosts since the spores are generally more resistant to stress than the growing bacterial cells.

While the processes and products of the present invention have been described primarily in reference to fusion products of bacteria capable of fixing nitrogen, it is to be understood that, with appropriate changes in the screening procedure, hybrids capable of producing other agricultural chemicals, such as insecticides, herbicides, antifungal agents, antibacterial agents, antiviral agents, plant growth regulators, and solubilized phosphates can be prepared in accordance with the present invention. Moreover, while the foregoing examples illustrate the inclusion of genes for production of one agricultural chemical in the hybrid, it is envisioned that genes for production of two or more compatible products can be included in hybrids found in accordance with the present invention. Thus, fusion of hybrids formed in accordance with the present invention with other agricultural-chemical-producing bacteria, initial fusion with organisms known to produce more than one agricultural chemical, or initial fusion conducted with more than one agricultural chemical producing bacterium would yield such multi-producing organisms. Similarly, the scope of applicable plant hosts can be expanded by fusions with two or more infecting bacteria.

**Claims**

**Claims for the following Contracting States : BE, CH, DE, FR, GB, IT, LI, LU, NL, SE**

1. A method of producing hybrid agricultural-chemical-producing bacteria capable of entering into non-pathogenic endosymbiotic relationships with a plant host comprising:

    (A) in any convenient order:

    (1) identifying said plant host;

    (2) identifying an infecting bacterium which infects said plant host;

    (3) selecting mutants of said infecting bacterium with one or more selectable traits in addition to ability to infect said host; and

    (4) selecting an agricultural-chemical-producing bacterium having one or more selectable traits;

    (B) forming fusion hybrids of said infecting bacterium and said agricultural-chemical-producing bacterium;

    (C) selecting serially from the products of the previously performed step or substep, and in any convenient order:

    (1) a bacterial subgroup comprising those hybrids having both at least one of said selectable traits of the agricultural-chemical-producing bacterium and at least one of said selectable traits of the infecting bacterium;

    (2) a bacterial subgroup comprising those hybrids which manifest the ability to interact with plant tissue in the manner in which said infecting bacterium interacts with plant tissue during the initial phase of infection in said plant host;

    (3) a bacterial subgroup comprising those hybrids which do not upon application to said host create manifestations of disease; and

    (4) a bacterial subgroup comprising those hybrids having the ability to produce said agricultural chemical if not previously selected for; and

    (D) selecting from the products of the last performed step of steps (C)(1) to (C)(4) those hybrids capable of improving the performance of said plant host when compared either by direct application of said agricultural chemical or by application of said agricultural-chemical-producing bacterium to said plant.

2. The method of Claim 1, wherein the selectable trait of the agricultural-chemical-producing bacteria selected for in step (C)(1) is the ability to produce said agricultural chemical.

3. The method of Claim 1, wherein the selectable trait of the agricultural-chemical-producing bacteria selected for in step (C)(1) is one or more selectable traits in addition to the ability to produce said agricultural chemical.

4. The method of Claim 1, wherein the further step of modifying said endosymbiotic agricultural-chemical-producing bacteria by natural or artificial genetic techniques to improve their performance as sources of said agricultural chemical for said plant host is included.

5. The method of Claim 1, wherein steps (C)(1) and/or (C)(2) are carried out two or more times.

6. The method of Claim 1, wherein the ability of step (C)(2) is the ability to bind to plant cells.

7. The method of Claim 1, wherein the ability of step (C)(2) is the ability to spread through the vascular system of said plant host.

8. The method of Claim 1, wherein the ability of step (C)(2) is the ability to live in the roots of the plant host without creating symptoms of disease.

9. The method of Claims 1 and 6, wherein the products of step (C) are further limited prior to step (D) by selection for further screening in accordance with this process of those hybrids which spread most readily throughout said plant host.

10. The method of Claim 1, wherein said agricultural-chemical-producing bacterium is a nitrogen-fixing bacterium.

11. The method of Claim 1, wherein said agricultural-chemical producing bacterium and said infecting bacterium are both gram-negative bacteria.

12. The method of Claim 1, wherein steps (C)(1) through (C)(4) are conducted in the order listed.

13. The method of Claim 1, wherein steps (C)(1) through (C)(3) are conducted in the order listed and step (C)(4) is omitted.

14. The method of Claim 1, wherein said fusion hybrids in step (B) are formed by protoplast fusion.

15. The method of Claim 1, wherein said fusion hybrids in step (B) are formed by spheroplast fusion.

16. A stable fusion hybrid bacterium having an agricultural-chemical-producing ability and being a non-pathogenic endosymbiont of a plant host, obtainable by any of the Claims 1 to 15.

17. A stable fusion hybrid bacterium according to Claim 16, wherein said infecting bacterium is of a genus selected from the group consisting of Agrobacterium, Erwinia, Pseudomonas, Xanthomonas or Azospirillum.

18. A stable fusion hybrid bacterium according to Claim 16, wherein said agricultural-chemical-producing bacterium is a nitrogen-fixing bacterium.

19. A stable fusion hybrid bacterium according to Claim 18, wherein said nitrogen-fixing bacterium is of the genus Azotobacter.

20. A stable fusion hybrid bacterium according to Claim 16, wherein said agricultural-chemical-producing bacterium is a bacterium having the ability to produce an anti-bacterial agent.

21. A stable fusion hybrid bacterium according to Claim 16, wherein said agricultural-chemical-producing bacterium is a bacterium having the ability to produce an antiviral agent.

25

**22.** A stable fusion hybrid bacterium according to Claim 16, wherein said agricultural-chemical-producing bacterium is a bacterium having the ability to produce an insecticide.

**23.** A stable fusion hybrid bacterium according to Claim 16, wherein said agricultural-chemical-producing bacterium is a bacterium having the ability to produce a nematocide.

**24.** A stable fusion hybrid bacterium according to Claim 16, wherein said agricultural-chemical-producing bacterium is a bacterium having the ability to produce a miticide.

**25.** A stable fusion hybrid bacterium according to Claim 16, wherein said agricultural-chemical-producing bacterium is a bacterium having the ability to produce a herbicide.

**26.** A stable fusion hybrid bacterium according to Claim 16, wherein said agricultural-chemical-producing bacterium is a bacterium having the ability to produce a fungicide.

**27.** A stable fusion hybrid bacterium according to any one of Claims 20, 21, 23, 24, 25 and 26, wherein said bacterium is of the genus Streptomyces.

**28.** A stable fusion hybrid bacterium according to Claim 16, wherein said agricultural-chemical-producing bacterium is a bacterium having the ability to solubilize bound phosphates.

**29.** A stable fusion hybrid bacterium according to Claim 22 or Claim 28, wherein said bacterium is of the genus Bacillus.

**30.** A stable fusion hybrid bacterium according to Claim 16, wherein said agricultural-chemical-producing bacterium is a bacterium having the ability to produce a plant growth regulator.

**31.** A stable fusion hybrid bacterium according to Claim 30, wherein said bacterium having the ability to produce a plant growth regulator is of the genus Pseudomonas.

**32.** A stable fusion hybrid bacterium according to Claim 16, wherein said agricultural-chemical-producing bacterium is a bacterium having the ability to produce chemicals selected from the group consisting of fragrances and antifeeding agents.

**33.** An agricultural seed product comprising crop plant seed, a biodegradable nutrient carrier material coated on said seed, and a stable fusion hybrid bacterium as claimed in Claim 16 associated with said carrier material.

**34.** An agricultural product comprising crop plant seed infected with a stable fusion hybrid bacterium as claimed in Claim 16.

**35.** An agricultural soil drench comprising a mixture of water and a stable fusion hybrid bacterium as claimed in Claim 16.

**36.** An agricultural product comprising a stable hybrid bacterium as claimed in Claim 16 in a horticulturally acceptable carrier suitable for injection into said plant host.

**Claim for the following Contracting State : AT**

**1.** A method of producing hybrid agricultural-chemical-producing bacteria capable of entering into non-pathogenic endosymbiotic relationships with a plant host comprising:
    (A) in any convenient order:
        (1) identifying said plant host;
        (2) identifying an infecting bacterium which infects said plant host;
        (3) selecting mutants of said infecting bacterium with one or more selectable traits in addition to ability to infect said host; and
        (4) selecting an agricultural-chemical-producing bacterium having one or more selectable traits;
    (B) forming fusion hybrids of said infecting bacterium and said agricultural-chemical-producing bacterium;

(C) selecting serially from the products of the previously performed step or substep, and in any convenient order:

(1) a bacterial subgroup comprising those hybrids having both at least one of said selectable traits of the agricultural-chemical-producing bacterium and at least one of said selectable traits of the infecting bacterium;

(2) a bacterial subgroup comprising those hybrids which manifest the ability to interact with plant tissue in the manner in which said infecting bacterium interacts with plant tissue during the initial phase of infection in said plant host;

(3) a bacterial subgroup comprising those hybrids which do not upon application to said host create manifestations of disease; and

(4) a bacterial subgroup comprising those hybrids having the ability to produce said agricultural chemical if not previously selected for; and

(D) selecting from the products of the last performed step of steps (C)(1) to (C)(4) those hybrids capable of improving the performance of said plant host when compared either by direct application of said agricultural chemical or by application of said agricultural-chemical-producing bacterium to said plant.

2. The method of Claim 1, wherein the selectable trait of the agricultural-chemical-producing bacteria selected for in step (C)(1) is the ability to produce said agricultural chemical.

3. The method of Claim 1, wherein the selectable trait of the agricultural-chemical-producing bacteria selected for in step (C)(1) is one or more selectable traits in addition to the ability to produce said agricultural chemical.

4. The method of Claim 1, wherein the further step of modifying said endosymbiotic agricultural-chemical-producing bacteria by natural or artificial genetic techniques to improve their performance as sources of said agricultural chemical for said plant host is included.

5. The method of Claim 1, wherein steps (C)(1) and/or (C)(2) are carried out two or more times.

6. The method of claim 1, wherein the ability of step (C)(2) is the ability to bind to plant cells.

7. The method of claim 1, wherein the ability of step (C)(2) is the ability to spread through the vascular system of said plant host.

8. The method of Claim 1, wherein the ability of step (C)(2) is the ability to live in the roots of the plant host without creating symptoms of disease.

9. The method of claims 1 and 6, wherein the products of step (C) are further limited prior to step (D) by selection for further screening in accordance with this process of those hybrids which spread most readily throughout said plant host.

10. The method of claim 1, wherein said agricultural-chemical-producing bacterium is a nitrogen-fixing bacterium.

11. The method of claim 1, wherein said agricultural-chemical producing bacterium and said infecting bacterium are both gram-negative bacteria.

12. The method of claim 1, wherein steps (C)(1) through (C)(4) are conducted in the order listed.

13. The method of claim 1, wherein steps (C)(1) through (C)(3) are conducted in the order listed and step (C)(4) is omitted.

14. The method of claim 1, wherein said fusion hybrids in step (B) are formed by protoplast fusion.

15. The method of Claim 1, wherein said fusion hybrids in step (B) are formed by spheroplast fusion.

16. A stable fusion hybrid bacterium having an agricultural-chemical-producing ability and being a non-pathogenic endosymbiont of a plant host, obtainable by any of the Claims 1 to 15.

17. A stable fusion hybrid bacterium according to claim 16, wherein said infecting bacterium is of a genus selected from the group consisting of Agrobacterium, Erwinia, Pseudomonas, Xanthomonas or Azospirillum.

18. A stable fusion hybrid bacterium according to Claim 16, wherein said agricultural-chemical-producing bacterium is a nitrogen-fixing bacterium.

19. A stable fusion hybrid bacterium according to claim 18, wherein said nitrogen-fixing bacterium is of the genus Azotobacter.

20. A stable fusion hybrid bacterium according to claim 16, wherein said agricultural-chemical-producing bacterium is a bacterium having the ability to produce an anti-bacterial agent.

21. A stable fusion hybrid bacterium according to claim 16, wherein said agricultural-chemical-producing bacterium is a bacterium having the ability to produce an antiviral agent.

22. A stable fusion hybrid bacterium according to Claim 16, wherein said agricultural-chemical-producing bacterium is a bacterium having the ability to produce an insecticide.

23. A stable fusion hybrid bacterium according to Claim 16, wherein said agricultural-chemical-producing bacterium is a bacterium having the ability to produce a nematocide.

24. A stable fusion hybrid bacterium according to claim 16, wherein said agricultural-chemical-producing bacterium is a bacterium having the ability to produce a miticide.

25. A stable fusion hybrid bacterium according to Claim 16, wherein said agricultural-chemical-producing bacterium is a bacterium having the ability to produce a herbicide.

26. A stable fusion hybrid bacterium according to Claim 16, wherein said agricultural-chemical-producing bacterium is a bacterium having the ability to produce a fungicide.

27. A stable fusion hybrid bacterium according to any one of Claims 20, 21, 23, 24, 25 and 26, wherein said bacterium is of the genus Streptomyces.

28. A stable fusion hybrid bacterium according to Claim 16, wherein said agricultural-chemical-producing bacterium is a bacterium having the ability to solubilize bound phosphates.

29. A stable fusion hybrid bacterium according to Claim 22 or Claim 28, wherein said bacterium is of the genus Bacillus.

30. A stable fusion hybrid bacterium according to Claim 16, wherein said agricultural-chemical-producing bacterium is a bacterium having the ability to produce a plant growth regulator.

31. A stable fusion hybrid bacterium according to Claim 30, wherein said bacterium having the ability to produce a plant growth regulator is of the genus Pseudomonas.

32. A stable fusion hybrid bacterium according to Claim 16, wherein said agricultural-chemical-producing bacterium is a bacterium having the ability to produce chemicals selected from the group consisting of fragrances and antifeeding agents.

33. An agricultural seed product comprising crop plant seed, a biodegradable nutrient carrier material coated on said seed, and a stable fusion hybrid bacterium as claimed in Claim 16 associated with said carrier material.

34. An agricultural product comprising crop plant seed infected with a stable fusion hybrid bacterium as

28

claimed in Claim 16.

**35.** An agricultural soil drench comprising a mixture of water and a stable fusion hybrid bacterium as claimed in Claim 16.

**36.** An agricultural product comprising a stable hybrid bacterium as claimed in Claim 16 in a horticulturally acceptable carrier suitable for injection into said plant host.

**37.** The method according to any of Claims 1 to 15, wherein said infecting bacterium is of a genus selected from the group consisting of Agrobacterium, Erwinia, Pseudomonas, Xanthomonas or Azospirillum.

**38.** The method according to Claim 10, wherein said nitrogen-fixing bacterium is of the genus Azotobacter.

**39.** The method according to any of Claims 1 to 15, wherein said agricultural-chemical-producing bacterium is a bacterium having the ability to produce an anti-bacterial agent.

**40.** The method according to any of Claims 1 to 15, wherein said agricultural-chemical-producing bacterium is a bacterium having the ability to produce an antiviral agent.

**41.** The method according to any of Claims 1 to 15, wherein said agricultural-chemical-producing bacterium is a bacterium having the ability to produce an insecticide.

**42.** The method according to any of Claims 1 to 15, wherein said agricultural-chemical-producing bacterium is a bacterium having the ability to produce a nematocide.

**43.** The method according to any of Claims 1 to 15, wherein said agricultural-chemical-producing bacterium is a bacterium having the ability to produce a miticide.

**44.** The method according to any of Claims 1 to 15, wherein said agricultural-chemical-producing bacterium is a bacterium having the ability to produce a herbicide.

**45.** The method according to any of Claims 1 to 15, wherein said agricultural-chemical-producing bacterium is a bacterium having the ability to produce a fungicide.

**46.** The method according to any of Claims 39, 40, 42, 43, 44 and 45, wherein said bacterium is of the genus Streptomyces.

**47.** The method according to any of Claims 1 to 15, wherein said agricultural-chemical-producing bacterium is a bacterium having the ability to solubilize bound phosphates.

**48.** The method according to Claim 41 or Claim 47, wherein said bacterium is of the genus Bacillus.

**49.** The method according to any of Claims 1 to 15, wherein said agricultural-chemical-producing bacterium is a bacterium having the ability to produce a plant growth regulator.

**50.** The method according to Claim 49, wherein said bacterium having the ability to produce a plant growth regulator is of the genus Pseudomonas.

**51.** The method according to any of Claims 1 to 15, wherein said agricultural-chemical-producing bacterium is a bacterium having the ability to produce chemicals selected from the group consisting of fragrances and antifeeding agents.

**Patentansprüche**
**Patentansprüche für folgende Vertragsstaaten : BE, CH, CE, FR, GB, IT, LI, LU, NL, SE**

**1.** Verfahren zur Herstellung hybrider Landwirtschaftschemikalien produzierender Bakterien, die in nichtpathogene, endosymbiotische Beziehungen mit einer Wirtspflanze treten können, umfassend:
(A) in beliebiger Reihenfolge:

EP 0 125 468 B1

(1) Identifizieren der Wirtspflanze;

(2) Identifizieren eines infizierenden Bakteriums, das die Wirtspflanze infiziert;

(3) Selektieren von Mutanten des infizierenden Bakteriums mit einer oder mehrerer selektierbaren Eigenschaften zusätzlich zu der Fähigkeit, die Wirtspflanze zu infizieren; und

(4) Selektieren eines Landwirtschaftschemikalien produzierenden Bakteriums mit einer oder mehreren selektierbaren Eigenschaften;

(B) Erzeugen von Fusionshybriden des infizierenden Bakteriums und des Landwirtschaftschemikalien produzierenden Bakteriums;

(C) nacheinander Selektieren aus den Produkten des vorher durchgeführten Schritts oder Teilschritts in beliebiger Reihenfolge:

(1) einer bakteriellen Untergruppe, die solche Hybride umfaßt, die mindestens eine der selektierbaren Eigenschaften des Landwirtschaftschemikalien produzierenden Bakteriums und mindestens eine der selektierbaren Eigenschaften des infizierenden Bakteriums besitzen;

(2) einer bakteriellen Untergruppe, die solche Hybride umfaßt, die die Fähigkeit manifestieren, mit Pflanzengewebe auf die gleiche Weise wechselzuwirken, wie das infizierende Bakterium mit Pflanzengewebe während der Anfangsphase der Infektion in der Wirtspflanze wechselwirkt;

(3) einer bakteriellen Untergruppe, die solche Hybride umfaßt, die bei der Anwendung auf den Wirt nicht Manifestationen einer Krankheit erzeugen; und

(4) einer bakteriellen Untergruppe, die solche Hybride umfaßt, die die Fähigkeit zum Herstellen der Landwirtschaftschemikalie besitzen, falls nicht zuvor darauf selektiert wurde; und

(D) Selektieren aus den Produkten des zuletzt durchgeführten Schritts der Schritte (C) (1) bis (C) (4) solcher Hybride, die die Leistung der Wirtspflanze verbessern können im Vergleich zu entweder direkter Anwendung der Landwirtschaftschemikalie oder Anwendung des Landwirtschaftschemikalien produzierenden Bakteriums auf die Pflanze.

2. Verfahren nach Anspruch 1, worin die im Schritt (C) (1) selektierte selektierbare Eigenschaft der Landwirtschaftschemikalien produzierenden Bakterien die Fähigkeit zum Herstellen der Landwirtschaftschemikalie ist.

3. Verfahren nach Anspruch 1, worin die im Schritt (C) (1) selektierte selektierbare Eigenschaft des Landwirtschaftschemikalien produzierenden Bakterien eine oder mehrere selektierbare Eigenschaften zusätzlich zu der Fähigkeit, die Landwirtschaftschemikalie herzustellen, sind.

4. Verfahren nach Anspruch 1, worin der weitere Schritt des Modifizierens der endosymbiotischen Landwirtschaftschemikalien produzierenden Bakterien durch natürliche oder künstliche genetische Techniken enthalten ist, um deren Leistungsfähigkeit als Quellen der Landwirtschaftschemikalie für die Wirtspflanze zu erhöhen.

5. Verfahren nach Anspruch 1, worin die Schritte (C) (1) und/oder (C) (2) zwei oder mehrmals ausgeführt werden.

6. Verfahren nach Anspruch 1, worin die Fähigkeit von Schritt (C) (2) die Fähigkeit ist, an Pflanzenzellen zu binden.

7. Verfahren nach Anspruch 1, worin die Fähigkeit von Schritt (C) (2) die Fähigkeit ist, sich über das Gefäßsystem der Wirtspflanze auszubreiten.

8. Verfahren nach Anspruch 1, worin die Fähigkeit von Schritt (C) (2) die Fähigkeit ist, in den Wurzeln der Wirtspflanze ohne Erzeugung von Krankheitssymptomen zu leben.

9. Verfahren nach den Ansprüchen 1 und 6, worin die Produkte von Schritt (C) weiterhin beschränkt werden vor Schritt (D) durch Selektion durch weiteres Absuchen gemäß diesem Verfahren auf solche Hybride, die sich am leichtesten über die Wirtspflanze ausbreiten.

10. Verfahren nach Anspruch 1, worin das Landwirtschaftschemikalien produzierende Bakterium ein Stickstoff bindendes Bakterium ist.

11. Verfahren nach Anspruch 1, worin das Landwirtschaftschemikalien produzierende Bakterium und das

30

infizierende Bakterium beide gram-negative Bakterien sind.

12. Verfahren nach Anspruch 1, worin Schritte (C) (1) bis (C) (4) in der angegebenen Reihenfolge durchgeführt werden.

13. Verfahren nach Anspruch 1, worin Schritte (C) (1) bis (C) (3) in der aufgeführten Reihenfolge durchgeführt werden und Schritt (C) (4) weggelassen wird.

14. Verfahren nach Anspruch 1, worin die Fusionshybride in Schritt (B) durch Protoplastenfusion erzeugt werden.

15. Verfahren nach Anspruch 1, worin die Fusionshybride in Schritt (B) durch Spheroplastenfusion erzeugt werden.

16. Stabiles Fusionshybridbakterium mit einer Landwirtschaftschemikalien produzierenden Fähigkeit und das ein nicht-pathogener Endosymbiont einer Wirtspflanze ist, erhältlich nach einem der Ansprüche 1 bis 15.

17. Stabiles Fusionshybridbakterium nach Anspruch 16, worin das infizierende Bakterium einer Gattung angehört, ausgewählt aus der Gruppe bestehend aus Agrobakterium, Erwinia, Pseudomonas, Xanthomonas oder Azospirillum.

18. Stabiles Fusionshybridbakterium nach Anspruch 16, worin das Landwirtschaftschemikalien produzierende Bakterium ein Stickstoff bindendes Bakterium ist.

19. Stabiles Fusionshybridbakterium nach Anspruch 18, worin das Stickstoff bindende Bakterium zu der Gattung Azotobacter gehört.

20. Stabiles Fusionshybridbakterium nach Anspruch 16, worin das Landwirtschaftschemikalien produzierende Bakterium ein Bakterium ist mit der Fähigkeit, ein antibakterielles Agenz zu bilden.

21. Stabiles Fusionshybridbakterium nach Anspruch 16, worin das Landwirtschaftschemikalien produzierende Bakterium ein Bakterium ist mit der Fähigkeit, ein antivirales Agens zu bilden.

22. Stabiles Fusionshybridbakterium nach Anspruch 16, worin das Landwirtschaftschemikalien produzierende Bakterium ein Bakterium ist mit der Fähigkeit, ein Insektizid zu bilden.

23. Stabiles Fusionshybridbakterium nach Anspruch 16, worin das Landwirtschaftschemikalien produzierende Bakterium ein Bakterium ist mit der Fähigkeit, ein Nematozid zu bilden.

24. Stabiles Fusionshybridbakterium nach Anspruch 16, worin das Landwirtschartschemikalien produzierende Bakterium ein Bakterium ist mit der Fähigkeit, ein Mitizid zu bilden.

25. Stabiles Fusionshybridbakterium nach Anspruch 16, worin das Landwirtschaftschemikalien produzierende Bakterium ein Bakterium ist mit der Fähigkeit, ein Herbizid zu bilden.

26. Stabiles Fusionshybridbakterium nach Anspruch 16, worin das Landwirtschaftschemikalien produzierende Bakterium ein Bakterium ist mit der Fähigkeit, ein Fungizid zu bilden.

27. Stabiles Fusionshybridbakterium nach einem der Ansprüche 20, 21, 23, 24, 25 und 26, worin das Bakterium zu der Gattung Streptomyces gehört.

28. Stabiles Fusionshybridbakterium nach Anspruch 16, worin das Landwirtschaftschemikalien produzierende Bakterium ein Bakterium ist mit der Fähigkeit, gebundene Phosphate löslich zu machen.

29. Stabiles Fusionshybridbakterium nach Anspruch 22 oder 28, worin das Bakterium zu der Gattung Bacillus gehört.

**30.** Stabiles Fusionshybridbakterium nach Anspruch 16, worin das Landwirtschaftschemikalien produzierende Bakterium ein Bakterium ist mit der Fähigkeit, einen Pflanzenwachstumsregulator zu bilden.

**31.** Stabiles Fusionshybridbakterium nach Anspruch 30, worin das Bakterium mit der Fähigkeit, einen Pflanzenwachstumsregulator zu bilden, zu der Gattung Pseudomonas gehört.

**32.** Stabiles Fusionshybridbakterium nach Anspruch 16, worin das Landwirtschaftschemikalien produzierende Bakterium ein Bakterium ist mit der Fähigkeit, Chemikalien zu produzieren, ausgewählt aus der Gruppe bestehend aus Aromastoffen und fraßverhindernden Agenzien.

**33.** Landwirtschaftliches Samenprodukt umfassend Pflanzensamen von Feldfrüchten, ein biologisch abbaubares Nährstoffträgermaterial, beschichtet auf den Samen, und ein stabiles Fusionshybridbakterium wie in Anspruch 16 beansprucht, assoziiert mit dem Trägermaterial.

**34.** Landwirtschaftliches Produkt umfassend Pflanzensamen von Feldfrüchten, infiziert mit einem stabilen Fusionshybridbakterium wie in Anspruch 16 beansprucht.

**35.** Landwirtschaftliche Bodenbeizflüssigkeit umfassend eine Mischung aus Wasser und ein stabiles Fusionshybridbakterium wie in Anspruch 16 beansprucht.

**36.** Landwirtschaftliches Produkt umfassend ein stabiles Hybridbakterium wie in Anspruch 16 beansprucht in einem gartenbauverträglichen Träger, geeignet zur Injektion in die Wirtspflanze.

**Patentansprüche für folgenden Vertragsstaat : AT**

**1.** Verfahren zur Herstellung hybrider Landwirtschaftschemikalien produzierender Bakterien, die in nichtpathogene, endosymbiotische Beziehungen mit einer Wirtspflanze treten können, umfassend:

(A) in beliebiger Reihenfolge:

(1) Identifizieren der Wirtspflanze;

(2) Identifizieren eines infizierenden Bakteriums, das die Wirtspflanze infiziert;

(3) Selektieren von Mutanten des infizierenden Bakteriums mit einer oder mehrerer selektierbaren Eigenschaften zusätzlich zu der Fähigkeit, die Wirtspflanze zu infizieren; und

(4) Selektieren eines Landwirtschaftschemikalien produzierenden Bakteriums mit einer oder mehreren selektierbaren Eigenschaften;

(B) Erzeugen von Fusionshybriden des infizierenden Bakteriums und des Landwirtschaftschemikalien produzierenden Bakteriums;

(C) nacheinander Selektieren aus den Produkten des vorher durchgeführten Schritts oder Teilschritts in beliebiger Reihenfolge:

(1) einer bakteriellen Untergruppe, die solche Hybride umfaßt, die mindestens eine der selektierbaren Eigenschaften des Landwirtschaftschemikalien produzierenden Bakteriums und mindestens eine der selektierbaren Eigenschaften des infizierenden Bakteriums besitzen;

(2) einer bakteriellen Untergruppe, die solche Hybride umfaßt, die die Fähigkeit manifestieren, mit Pflanzengewebe auf die gleiche Weise wechselzuwirken, wie das infizierende Bakterium mit Pflanzengewebe während der Anfangsphase der Infektion in der Wirtspflanze wechselwirkt;

(3) einer bakteriellen Untergruppe, die solche Hybride umfaßt, die bei der Anwendung auf den Wirt nicht Manifestationen einer Krankheit erzeugen; und

(4) einer bakteriellen Untergruppe, die solche Hybride umfaßt, die die Fähigkeit zum Herstellen der Landwirtschaftschemikalie besitzen, falls nicht zuvor darauf selektiert wurde; und

(D) Selektieren aus den Produkten des zuletzt durchgeführten Schritts der Schritte (C) (1) bis (C) (4) solcher Hybride, die die Leistung der Wirtspflanze verbessern können im Vergleich zu entweder direkter Anwendung der Landwirtschaftschemikalie oder Anwendung des Landwirtschaftschemikalien produzierenden Bakteriums auf die Pflanze.

**2.** Verfahren nach Anspruch 1, worin die im Schritt (C) (1) selektierte selektierbare Eigenschaft der Landwirtschaftschemikalien produzierenden Bakterien die Fähigkeit zum Herstellen der Landwirtschaftschemikalie ist.

**3.** Verfahren nach Anspruch 1, worin die im Schritt (C) (1) selektierte selektierbare Eigenschaft des Landwirtschaftschemikalien produzierenden Bakterien eine oder mehrere selektierbare Eigenschaften zusätzlich zu der Fähigkeit, die Landwirtschaftschemikalie herzustellen, sind.

4. Verfahren nach Anspruch 1, worin der weitere Schritt des Modifizierens der endosymbiotischen Landwirtschaftschemikalien produzierenden Bakterien durch natürliche oder künstliche genetische Techniken enthalten ist, um deren Leistungsfähigkeit als Quellen der Landwirtschaftschemikalie für die Wirtspflanze zu erhöhen.

5. Verfahren nach Anspruch 1, worin die Schritte (C) (1) und/oder (C) (2) zwei oder mehrmals ausgeführt werden.

6. Verfahren nach Anspruch 1, worin die Fähigkeit von Schritt (C) (2) die Fähigkeit ist, an Pflanzenzellen zu binden.

7. Verfahren nach Anspruch 1, worin die Fähigkeit von Schritt (C) (2) die Fähigkeit ist, sich über das Gefäßsystem der Wirtspflanze auszubreiten.

8. Verfahren nach Anspruch 1, worin die Fähigkeit von Schritt (C) (2) die Fähigkeit ist, in den Wurzeln der Wirtspflanze ohne Erzeugung von Krankheitssymptomen zu leben.

9. Verfahren nach den Ansprüchen 1 und 6, worin die Produkte von Schritt (C) weiterhin beschränkt werden vor Schritt (D) durch Selektion durch weiteres Absuchen gemäß diesem Verfahren auf solche Hybride, die sich am leichtestens über die Wirtspflanze ausbreiten.

10. Verfahren nach Anspruch 1, worin das Landwirtschaftschemikalien produzierende Bakterium ein Stickstoff bindendes Bakterium ist.

11. Verfahren nach Anspruch 1, worin das Landwirtschaftschemikalien produzierende Bakterium und das infizierende Bakterium beide gram-negative Bakterien sind.

12. Verfahren nach Anspruch 1, worin Schritte (C) (1) bis (C) (4) in der angegebenen Reihenfolge durchgeführt werden.

13. Verfahren nach Anspruch 1, worin Schritte (C) (1) bis (C) (3) in der aufgeführten Reihenfolge durchgeführt werden und Schritt (C) (4) weggelassen wird.

14. Verfahren nach Anspruch 1, worin die Fusionshybride in Schritt (B) durch Protoplastenfusion erzeugt werden.

15. Verfahren nach Anspruch 1, worin die Fusionshybride in Schritt (B) durch Spheroplastenfusion erzeugt werden.

16. Stabiles Fusionshybridbakterium mit einer Landwirtschaftschemikalien produzierenden Fähigkeit und das ein nicht-pathogener Endosymbiont einer Wirtspflanze ist, erhältlich nach einem der Ansprüche 1 bis 15.

17. Stabiles Fusionshybridbakterium nach Anspruch 16, worin das infizierende Bakterium einer Gattung angehört, ausgewählt aus der Gruppe bestehend aus Agrobakterium, Erwinia, Pseudomonas, Xanthomonas oder Azospirillum.

18. Stabiles Fusionshybridbakterium nach Anspruch 16, worin das Landwirtschaftschemikalien produzierende Bakterium ein Stickstoff bindendes Bakterium ist.

19. Stabiles Fusionshybridbakterium nach Anspruch 18, worin das Stickstoff bindende Bakterium Zu der Gattung Azotobacter gehört.

20. Stabiles Fusionshybridbakterium nach Anspruch 16, worin das Landwirtschaftschemikalien produzierende Bakterium ein Bakterium ist mit der Fähigkeit, ein antibakterielles Agenz zu bilden.

EP 0 125 468 B1

**21.** Stabiles Fusionshybridbakterium nach Anspruch 16, worin das Landwirtschaftschemikalien produzierende Bakterium ein Bakterium ist mit der Fähigkeit, ein antivirales Agens Zu bilden.

**22.** Stabiles Fusionshybridbakterium nach Anspruch 16, worin das Landwirtschaftschemikalien produzierende Bakterium ein Bakterium ist mit der Fähigkeit, ein Insektizid zu bilden.

**23.** Stabiles Fusionshybridbakterium nach Anspruch 16, worin das Landwirtschaftschemikalien produzierende Bakterium ein Bakterium ist mit der Fähigkeit, ein Nematozid zu bilden.

**24.** Stabiles Fusionshybridbakterium nach Anspruch 16, worin das Landwirtschaftschemikalien produzierende Bakterium ein Bakterium ist mit der Fähigkeit, ein Mitizid zu bilden.

**25.** Stabiles Fusionshybridbakterium nach Anspruch 16, worin das Landwirtschaftschemikalien produzierende Bakterium ein Bakterium ist mit der Fähigkeit, ein Herbizid zu bilden.

**26.** Stabiles Fusionshybridbakterium nach Anspruch 16, worin das Landwirtschaftschemikalien produzierende Bakterium ein Bakterium ist mit der Fähigkeit, ein Fungizid zu bilden.

**27.** Stabiles Fusionshybridbakterium nach einem der Ansprüche 20, 21, 23, 24, 25 und 26, worin das Bakterium zu der Gattung Streptomyces gehört.

**28.** Stabiles Fusionshybridbakterium nach Anspruch 16, worin das Landwirtschaftschemikalien produzierende Bakterium ein Bakterium ist mit der Fähigkeit, gebundene Phosphate löslich zu machen.

**29.** Stabiles Fusionshybridbakterium nach Anspruch 22 oder 28, worin das Bakterium zu der Gattung Bacillus gehört.

**30.** Stabiles Fusionshybridbakterium nach Anspruch 16, worin das Landwirtschaftschemikalien produzierende Bakterium ein Bakterium ist mit der Fähigkeit, einen Pflanzenwachstumsregulator zu bilden.

**31.** Stabiles Fusionshybridbakterium nach Anspruch 30, worin das Bakterium mit der Fähigkeit, einen Pflanzenwachstumsregulator zu bilden, zu der Gattung Pseudomonas gehört.

**32.** Stabiles Fusionshybridbakterium nach Anspruch 16, worin das Landwirtschaftschemikalien produzierende Bakterium ein Bakterium ist mit der Fähigkeit, Chemikalien zu produzieren, ausgewählt aus der Gruppe bestehend aus Aromastoffen und fraßverhindernden Agenzien.

**33.** Landwirtschaftliches Samenprodukt umfassend Pflanzensamen von Feldfrüchten, ein biologisch abbaubares Nährstoffträgermaterial, beschichtet auf den Samen, und ein stabiles Fusionshybridbakterium wie in Anspruch 16 beansprucht, assoziiert mit dem Trägermaterial.

**34.** Landwirtschaftliches Produkt umfassend Pflanzensamen von Feldfrüchten, infiziert mit einem stabilen Fusionshybridbakterium wie in Anspruch 16 beansprucht.

**35.** Landwirtschaftliche Bodenbeizflüssigkeit umfassend eine Mischung aus Wasser und ein stabiles Fusionshybridbakterium wie in Anspruch 16 beansprucht.

**36.** Landwirtschaftliches Produkt umfassend ein stabiles Hybridbakterium wie in Anspruch 16 beansprucht in einem gartenbauverträglichen Träger, geeignet zur Injektion in die Wirtspflanze.

**37.** Verfahren gemäß einem der Ansprüche 1 bis 15, worin das infizierende Bakterium einer Gattung angehört, ausgewählt aus der Gruppe, bestehend aus Agrobakterium, Erwinia, Pseudomonas, Xanthomonas oder Azospirillum.

**38.** Verfahren gemäß Anspruch 10, worin das stickstoffbindende Bakterium zu der Gattung Azotobacter gehört.

**39.** Verfahren gemäß einem der Ansprüche 1 bis 15, worin das Landwirtschaftschemikalien produzierende

34

EP 0 125 468 B1

Bakterium ein Bakterium ist mit der Fähigkeit, ein antibakterielles Agens zu bilden.

**40.** Verfahren gemäß einem der Ansprüche 1 bis 15, worin das Landwirtschaftschemikalien produzierende Bakterium ein Bakterium ist mit der Fähigkeit, ein antivirales Agens zu bilden.

**41.** Verfahren gemäß einem der Ansprüche 1 bis 15, worin das Landwirtschaftschemikalien produzierende Bakterium ein Bakterium ist mit der Fähigkeit, ein Insektizid zu bilden.

**42.** Verfahren gemäß einem der Ansprüche 1 bis 15, worin das Landwirtschaftschemikalien produzierende Bakterium ein Bakterium ist mit der Fähigkeit, ein Nematozid zu bilden.

**43.** Verfahren gemäß einem der Ansprüche 1 bis 15, worin das Landwirtschaftschemikalien produzierende Bakterium ein Bakterium ist mit der Fähigkeit, ein Mitizid zu bilden.

**44.** Verfahren gemäß einem der Ansprüche 1 bis 15, worin das Landwirtschaftschemikalien produzierende Bakterium ein Bakterium ist mit der Fähigkeit, ein Herbizid zu bilden.

**45.** Verfahren gemäß einem der Ansprüche 1 bis 15, worin das Landwirtschaftschemikalien produzierende Bakterium ein Bakterium ist mit der Fähigkeit, ein Fungizid zu bilden.

**46.** Verfahren gemäß einem der Ansprüche 39, 40, 42, 43, 44 und 45, worin das Bakterium zu der Gattung <u>Streptomyces</u> gehört.

**47.** Verfahren gemäß einem der Ansprüche 1 bis 15, worin das Landwirtschaftschemikalien produzierende Bakterium ein Bakterium ist mit der Fähigkeit, gebundene Phosphate löslich zu machen.

**48.** Verfahren gemäß einem der Ansprüche 41 oder 47, worin das Bakterium zu der Gattung <u>Bazillus</u> gehört.

**49.** Verfahren gemäß einem der Ansprüche 1 bis 15, worin das Landwirtschaftschemikalien produzierende Bakterium ein Bakterium ist mit der Fähigkeit, einen Pflanzenwachstumsregulator zu bilden.

**50.** Verfahren gemäß Anspruch 49, worin das Bakterium mit der Fähigkeit, einen Pflanzenwachstumsregulator zu bilden, zu der Gattung <u>Pseudomonas</u> gehört.

**51.** Verfahren gemäß einem der Ansprüche 1 bis 15, worin das Landwirtschaftschemikalien produzierende Bakterium ein Bakterium ist mit der Fähigkeit, Chemikalien zu produzieren, ausgewählt aus der Gruppe bestehend aus Aromastoffen und fraßverhindernden Agenzien.

## Revendications

**Revendications pour les Etats contractants suivants: BE, CH, DE, FR, GB, IT, LI, LU, NL, SE**

**1.** Procédé de production de bactéries hybrides productrices de produits chimiques agricoles capables d'entrer dans des relations endosymbiotiques non-pathogènes avec un hôte végétal comprenant :

(A) dans n'importe quel ordre commode :

(1) l'identification dudit hôte végétal ;

(2) l'identification d'une bactérie infectante qui infecte ledit hôte végétal ;

(3) la sélection de mutants de ladite bactérie infectante avec une ou plusieurs caractéristiques sélectionnables s'ajoutant à l'aptitude à infecter ledit hôte ; et

(4) la sélection d'une bactérie productrice de produits chimiques agricoles ayant une ou plusieurs caractéristiques sélectionnables ;

(B) la formation d'hybrides de fusion de ladite bactérie infectante et de ladite bactérie productrice de produits chimiques agricoles ;

(C) la sélection en série à partir des produits de l'étape ou sous-étape antérieurement réalisée, et dans n'importe quel ordre commode :

(1) d'un sous-groupe bactérien comprenant les hybrides ayant à la fois au moins une desdites caractéristiques sélectionnables de la bactérie productrice de produits chimiques agricoles et au moins une desdites caractéristiques sélectionnables de la bactérie infectante ;

35

(2) d'un sous-groupe bactérien comprenant les hybrides qui manifestent l'aptitude à interagir avec le tissu végétal de la manière dont ladite bactérie infectante interagit avec le tissu végétal pendant la phase initiale de l'infection chez ledit hôte végétal ;

(3) d'un sous-groupe bactérien comprenant les hybrides qui après application au dit hôte ne font pas apparaître de manifestations maladives ; et

(4) d'un sous-groupe bactérien comprenant les hybrides qui ont l'aptitude à produire ledit produit chimique agricole s'il n'a pas été préalablement sélectionné ; et

(D) la sélection à partir des produits de la dernière étape réalisée parmi les étapes (C)(1) à (C)(4), des hybrides capables d'améliorer la performance dudit hôte végétal lorsqu'on compare soit par application directe dudit produit chimique agricole, soit par application de ladite bactérie productrice de produit chimique agricole à ladite plante.

2. Procédé selon la revendication 1, dans lequel la caractéristique sélectionnable des bactéries productrices de produits chimiques agricoles sélectionnées dans l'étape (C)(1) est l'aptitude à produit ledit produit chimique agricole.

3. Procédé selon la revendication 1, dans lequel la caractéristique sélectionnable des bactéries productrices de produits chimiques agricoles sélectionnées dans l'étape (C)(1) est une ou plusieurs caractéristiques sélectionnables s'ajoutant à l'aptitude à produire ledit produit chimique agricole.

4. Procédé selon la revendication 1, dans lequel l'étape ultérieure de modification desdites bactéries endosymbiotiques productrices de produits chimiques agricoles, par des techniques génétiques naturelles ou artificielles, pour améliorer leur performance comme sources dudit produit chimique agricole pour ledit hôte végétal, est incluse.

5. Procédé selon la revendication 1, dans lequel les étapes (C)(1) et/ou (C)(2) sont effectuées deux ou plusieurs fois.

6. Procédé selon la revendication 1, dans lequel l'aptitude de l'étape (C)(2) est l'aptitude à se lier à des cellules végétales.

7. Procédé selon la revendication 1, dans lequel l'aptitude de l'étape (C)(2) est l'aptitude à se répandre à travers le système vasculaire dudit hôte végétal.

8. Procédé selon la revendication 1, dans lequel l'aptitude de l'étape (C)(2) est l'aptitude à vivre dans les racines de l'hote végétal sans créer de symptômes de maladie.

9. Procédé selon les revendications 1 et 6, dans lequel les produits de l'étape (C) sont encore limités avant l'étape (D) par une sélection en vue d'un criblage ultérieur, selon ce procédé, des hybrides qui se répandent le plus facilement à travers ledit hôte végétal.

10. Procédé selon la revendication 1, dans lequel ladite bactérie productrice de produit chimique agricole est une bactérie fixant l'azote.

11. Procédé selon la revendication 1, dans lequel ladite bactérie productrice de produits chimiques agricoles et ladite bactérie infectante sont toutes deux des bactéries gram-négatives.

12. Procédé selon la revendication 1, dans lequel les étapes (C)(1) à (C)(4) sont conduites dans l'ordre énuméré.

13. Procédé selon la revendication 1, dans lequel les étapes (C)(1) à (C)(3) sont conduites dans l'ordre énuméré et l'étape (C)(4) est omise.

14. Procédé selon la revendication 1, dans lequel lesdits hybrides de fusion de l'étape (B) sont formés par fusion de protoplastes.

15. Procédé selon la revendication 1, dans lequel lesdits hybrides de fusion de l'étape (B) sont formés par fusion de sphéroplastes.

**16.** Bactérie hybride de fusion stable ayant une aptitude à produire des produits chimiques agricoles et qui est un produit d'endosymbiose non-pathogène d'un hôte végétal, que l'on peut obtenir par l'une quelconque des revendications 1 à 15.

**17.** Bactérie hybride de fusion stable selon la revendication 16, dans laquelle ladite bactérie infectante est d'un genre sélectionné dans le groupe constitué par Agrobacterium, Erwinia, Pseudomonas, Xanthomonas ou Azospirillum.

**18.** Bactérie hybride de fusion stable selon la revendication 16, dans laquelle ladite bactérie productrice de produits chimiques agricoles est une bactérie fixant l'azote.

**19.** Bactérie hybride de fusion stable selon la revendication 18, dans laquelle ladite bactérie fixant l'azote est du genre Azotobacter.

**20.** Bactérie hybride de fusion stable selon la revendication 16, dans laquelle ladite bactérie productrice de produits chimiques agricoles est une bactérie capable de produire un agent antibactérien.

**21.** Bactérie hybride de fusion stable selon la revendication 16, dans laquelle ladite bactérie productrice de produits chimiques agricoles est une bactérie capable de produire un agent antiviral.

**22.** Bactérie hybride de fusion stable selon la revendication 16, dans laquelle ladite bactérie productrice de produits chimiques agricoles est une bactérie capable de produire un insecticide.

**23.** Bactérie hybride de fusion stable selon la revendication 16, dans laquelle ladite bactérie productrice de produits chimiques agricoles est une bactérie capable de produire un nématocide.

**24.** Bactérie hybride de fusion stable selon la revendication 16, dans laquelle ladite bactérie productrice de produits chimiques agricoles est une bactérie capable de produire un miticide.

**25.** Bactérie hybride de fusion stable selon la revendication 16, dans laquelle ladite bactérie productrice de produits chimiques agricoles est une bactérie capable de produire un herbicide.

**26.** Bactérie hybride de fusion stable selon la revendication 16, dans laquelle ladite bactérie productrice de produits chimiques agricoles est une bactérie capable de produire un fongicide.

**27.** Bactérie hybride de fusion stable selon l'une quelconque des revendications 20, 21, 23, 24, 25 et 26, dans laquelle ladite bactérie est du genre Streptomyces.

**28.** Bactérie hybride de fusion stable selon la revendication 16, dans laquelle ladite bactérie productrice de produits chimiques agricoles est une bactérie capable de solubiliser les phosphates liés.

**29.** Bactérie hybride de fusion stable selon la revendication 22 ou la revendication 28, dans laquelle ladite bactérie est du genre Bacillus.

**30.** Bactérie hybride de fusion stable selon la revendication 16, dans laquelle ladite bactérie productrice de produits chimiques agricoles est une bactérie capable de produire un régulateur de la croissance des plantes.

**31.** Bactérie hybride de fusion stable selon la revendication 30, dans laquelle ladite bactérie capable de produire un régulateur de la croissance des plantes est du genre Pseudomonas.

**32.** Bactérie hybride de fusion stable selon la revendication 16, dans laquelle ladite bactérie productrice de produits chimiques agricoles est une bactérie capable de produire des produits chimiques choisis dans le groupe constitué par les parfums et les agents antiappétence.

**33.** Produit d'ensemencement agricole comprenant une semence de plante cultivée, une matière support nutritive biodégradable déposée sur ladite semence, et une bactérie hybride de fusion stable selon la revendication 16 associée à ladite matière support.

**34.** Produit agricole comprenant une semence de plante cultivée infectée avec une bactérie hybride de fusion stable selon la revendication 16.

**35.** Pose médicamenteuse pour terre agricole comprenant un mélange d'eau et d'une bactérie hybride de fusion stable selon la revendication 16.

**36.** Produit agricole comprenant une bactérie hybride stable selon la revendication 16, dans un support acceptable en horticulture approprié à l'injection chez ledit hôte végétal.

**Revendications pour l'Etat contractant suivant : AT**

**1.** Procédé de production de bactéries hybrides productrices de produits chimiques agricoles capables d'entrer dans des relations endosymbiotiques non-pathogènes avec un hôte végétal comprenant :
(A) dans n'importe quel ordre commode :
(1) l'identification dudit hôte végétal ;
(2) l'identification d'une bactérie infectante qui infecte ledit hôte végétal ;
(3) la sélection de mutants de ladite bactérie infectante avec une ou plusieurs caractéristiques sélectionnables s'ajoutant à l'aptitude à infecter ledit hôte ; et
(4) la sélection d'une bactérie productrice de produits chimiques agricoles ayant une ou plusieurs caractéristiques sélectionnables ;
(B) la formation d'hybrides de fusion de ladite bactérie infectante et de ladite bactérie productrice de produits chimiques agricoles ;
(C) la sélection en série à partir des produits de l'étape ou sous-étape antérieurement réalisée, et dans n'importe quel ordre commode :
(1) d'un sous-groupe bactérien comprenant les hybrides ayant à la fois au moins une desdites caractéristiques sélectionnables de la bactérie productrice de produits chimiques agricoles et au moins une desdites caractéristiques sélectionnables de la bactérie infectante ;
(2) d'un sous-groupe bactérien comprenant les hybrides qui manifestent l'aptitude à interagir avec le tissu végétal de la manière dont ladite bactérie infectante interagit avec le tissu végétal pendant la phase initiale de l'infection chez ledit hôte végétal ;
(3) d'un sous-groupe bactérien comprenant les hybrides qui après application au dit hôte ne font pas apparaître de manifestations maladives ; et
(4) d'un sous-groupe bactérien comprenant les hybrides qui ont l'aptitude à produire ledit produit chimique agricole s'il n'a pas été préalablement sélectionné ; et
(D) la sélection à partir des produits de la dernière étape réalisée parmi les étapes (C)(1) à (C)(4), des hybrides capables d'améliorer la performance dudit hôte végétal lorsqu'on compare soit par application directe dudit produit chimique agricole, soit par application de ladite bactérie productrice de produit chimique agricole à ladite plante.

**2.** Procédé selon la revendication 1, dans lequel la caractéristique sélectionnable des bactéries productrices de produits chimiques agricoles sélectionnées dans l'étape (C)(1) est l'aptitude à produit ledit produit chimique agricole.

**3.** Procédé selon la revendication 1, dans lequel la caractéristique sélectionnable des bactéries productrices de produits chimiques agricoles sélectionnées dans l'étape (C)(1) est une ou plusieurs caractéristiques sélectionnables s'ajoutant à l'aptitude à produire ledit produit chimique agricole.

**4.** Procédé selon la revendication 1, dans lequel l'étape ultérieure de modification desdites bactéries endosymbiotiques productrices de produits chimiques agricoles, par des techniques génétiques naturelles ou artificielles, pour améliorer leur performance comme sources dudit produit chimique agricole pour ledit hôte végétal, est incluse.

**5.** Procédé selon la revendication 1, dans lequel les étapes (C)(1) et/ou (C)(2) sont effectuées deux ou plusieurs fois.

**6.** Procédé selon la revendication 1, dans lequel l'aptitude de l'étape (C)(2) est l'aptitude à se lier à des cellules végétales.

**7.** Procédé selon la revendication 1, dans lequel l'aptitude de l'étape (C)(2) est l'aptitude à se répandre à travers le système vasculaire dudit hôte végétal.

**8.** Procédé selon la revendication 1, dans lequel l'aptitude de l'étape (C)(2) est l'aptitude à vivre dans les racines de l'hote végétal sans créer de symptômes de maladie.

**9.** Procédé selon les revendications 1 et 6, dans lequel les produits de l'étape (C) sont encore limités avant l'étape (D) par une sélection en vue d'un criblage ultérieur, selon ce procédé, des hybrides qui se répandent le plus facilement à travers ledit hôte végétal.

**10.** Procédé selon la revendication 1, dans lequel ladite bactérie productrice de produit chimique agricole est une bactérie fixant l'azote.

**11.** Procédé selon la revendication 1, dans lequel ladite bactérie productrice de produits chimiques agricoles et ladite bactérie infectante sont toutes deux des bactéries gram-négatives.

**12.** Procédé selon la revendication 1, dans lequel les étapes (C)(1) à (C)(4) sont conduites dans l'ordre énuméré.

**13.** Procédé selon la revendication 1, dans lequel les étapes (C)(1) à (C)(3) sont conduites dans l'ordre énuméré et l'étape (C)(4) est omise.

**14.** Procédé selon la revendication 1, dans lequel lesdits hybrides de fusion de l'étape (B) sont formés par fusion de protoplastes.

**15.** Procédé selon la revendication 1, dans lequel lesdits hybrides de fusion de l'étape (B) sont formés par fusion de sphéroplastes.

**16.** Bactérie hybride de fusion stable ayant une aptitude à produire des produits chimiques agricoles et qui est un produit d'endosymbiose non-pathogène d'un hôte végétal, que l'on peut obtenir par l'une quelconque des revendications 1 à 15.

**17.** Bactérie hybride de fusion stable selon la revendication 16, dans laquelle ladite bactérie infectante est d'un genre sélectionné dans le groupe constitué par Agrobacterium, Erwinia, Pseudomonas, Xanthomonas ou Azospirillum.

**18.** Bactérie hybride de fusion stable selon la revendication 16, dans laquelle ladite bactérie productrice de produits chimiques agricoles est une bactérie fixant l'azote.

**19.** Bactérie hybride de fusion stable selon la revendication 18, dans laquelle ladite bactérie fixant l'azote est du genre Azotobacter.

**20.** Bactérie hybride de fusion stable selon la revendication 16, dans laquelle ladite bactérie productrice de produits chimiques agricoles est une bactérie capable de produire un agent antibactérien.

**21.** Bactérie hybride de fusion stable selon la revendication 16, dans laquelle ladite bactérie productrice de produits chimiques agricoles est une bactérie capable de produire un agent antiviral.

**22.** Bactérie hybride de fusion stable selon la revendication 16, dans laquelle ladite bactérie productrice de produits chimiques agricoles est une bactérie capable de produire un insecticide.

**23.** Bactérie hybride de fusion stable selon la revendication 16, dans laquelle ladite bactérie productrice de produits chimiques agricoles est une bactérie capable de produire un nématocide.

**24.** Bactérie hybride de fusion stable selon la revendication 16, dans laquelle ladite bactérie productrice de produits chimiques agricoles est une bactérie capable de produire un miticide.

**25.** Bactérie hybride de fusion stable selon la revendication 16, dans laquelle ladite bactérie productrice de produits chimiques agricoles est une bactérie capable de produire un herbicide.

26. Bactérie hybride de fusion stable selon la revendication 16, dans laquelle ladite bactérie productrice de produits chimiques agricoles est une bactérie capable de produire un fongicide.

27. Bactérie hybride de fusion stable selon l'une quelconque des revendications 20, 21, 23, 24, 25 et 26, dans laquelle ladite bactérie est du genre Streptomyces.

28. Bactérie hybride de fusion stable selon la revendication 16, dans laquelle ladite bactérie productrice de produits chimiques agricoles est une bactérie capable de solubiliser les phosphates liés.

29. Bactérie hybride de fusion stable selon la revendication 22 ou la revendication 28, dans laquelle ladite bactérie est du genre Bacillus.

30. Bactérie hybride de fusion stable selon la revendication 16, dans laquelle ladite bactérie productrice de produits chimiques agricoles est une bactérie capable de produire un régulateur de la croissance des plantes.

31. Bactérie hybride de fusion stable selon la revendication 30, dans laquelle ladite bactérie capable de produire un régulateur de la croissance des plantes est du genre Pseudomonas.

32. Bactérie hybride de fusion stable selon la revendication 16, dans laquelle ladite bactérie productrice de produits chimiques agricoles est une bactérie capable de produire des produits chimiques choisis dans le groupe constitué par les parfums et les agents antiappétence.

33. Produit d'ensemencement agricole comprenant une semence de plante cultivée, une matière support nutritive biodégradable déposée sur ladite semence, et une bactérie hybride de fusion stable selon la revendication 16 associée à ladite matière support.

34. Produit agricole comprenant une semence de plante cultivée infectée avec une bactérie hybride de fusion stable selon la revendication 16.

35. Dose médicamenteuse pour terre agricole comprenant un mélange d'eau et d'une bactérie hybride de fusion stable selon la revendication 16.

36. Produit agricole comprenant une bactérie hybride stable selon la revendication 16, dans un support acceptable en horticulture approprié à l'injection chez ledit hôte végétal.

37. Procédé selon l'une quelconque des revendications 1 à 15, dans lequel ladite bactérie infectante est d'un genre choisi dans le groupe constitué par Agrobacterium, Erwinia, Pseudomonas, Xanthomonas ou Azospirillum.

38. Procédé selon la revendication 10, dans lequel ladite bactérie fixant l'azote est du genre Azotobacter.

39. Procédé selon l'une quelconque des revendications 1 à 15, dans lequel ladite bactérie productrice de produits chimiques agricoles est une bactérie capable de produire un agent antibactérien.

40. Procédé selon l'une quelconque des revendications 1 à 15, dans lequel ladite bactérie productrice de produits chimiques agricoles est une bactérie capable de produire un agent antiviral.

41. Procédé selon l'une quelconque des revendications 1 à 15, dans lequel ladite bactérie productrice de produits chimiques agricoles est une bactérie capable de produire un insecticide.

42. Procédé selon l'une quelconque des revendications 1 à 15, dans lequel ladite bactérie productrice de produits chimiques agricoles est une bactérie capable de produire un nématocide.

43. Procédé selon l'une quelconque des revendications 1 à 15, dans lequel ladite bactérie productrice de produits chimiques agricoles est une bactérie capable de produire un miticide.

**44.** Procédé selon l'une quelconque des revendications 1 à 15, dans lequel ladite bactérie productrice de produits chimiques agricoles est une bactérie capable de produire un herbicide.

**45.** Procédé selon l'une quelconque des revendications 1 à 15, dans lequel ladite bactérie productrice de produits chimiques agricoles est une bactérie capable de produire un fongicide.

**46.** Procédé selon l'une quelconque des revendications 39, 40, 42, 43, 44 et 45, dans lequel ladite bactérie est du genre Streptomyces.

**47.** Procédé selon l'une quelconque des revendications 1 à 15, dans lequel ladite bactérie productrice de produits chimiques agricoles est une bactérie capable de solubiliser les phosphates liés.

**48.** Procédé selon la revendication 41 ou la revendication 47, dans lequel ladite bactérie est du genre Bacillus.

**49.** Procédé selon l'une quelconque des revendications 1 à 15, dans lequel ladite bactérie productrice de produits chimiques agricoles est une bactérie capable de produire un régulateur de la croissance des plantes.

**50.** Procédé selon la revendication 49, dans lequel ladite bactérie capable de produire un régulateur de la croissance des plantes est du genre Pseudomonas.

**51.** Procédé selon l'une quelconque des revendications 1 à 15, dans lequel ladite bactérie productrice de produits chimiques agricoles est une bactérie capable de produire des produits chimiques choisis dans le groupe constitué par les parfums et les agents antiappétence.